# EUROPEAN PATENT APPLICATION

(11) **EP 3 508 588 A1**
(43) Date of publication of application: **10.07.2019**
(21) Application number: 17846727.0
(22) Date of filing: 01.09.2017
(51) Int. Cl.: C12Q 1/68, C12N 9/16, C12N 15/09, C12Q 1/34, C12M 1/34

(54) **METHOD FOR AMPLIFYING METHYLATED DNA, METHOD FOR DETERMINING OF METHYLATION OF DNA, AND METHOD FOR DETERMINING OCCURRENCE OF CANCER**

(30) Priority: 02.09.2016 JP 2016171550
(71) Applicant: Fujifilm Wako Pure Chemical Corporation, Osaka-shi, Osaka 540-8605 (JP)
(72) Inventor: HAYASHIDA Yukinobu, Amagasaki-shi Hyogo 661-0963 (JP)
(74) Representative: Winter, Brandl, Fürniss, Hübner, Röss, Kaiser, Polte - Partnerschaft mbB
(86) International application number: PCT/JP2017/031670
(87) International publication number: WO 2018/043724

(57) **Abstract**

An object of the present invention is to provide a method capable of conveniently carrying out amplification of methylated DNA, and determination of methylation of DNA and determination of the occurrence of cancer using the amplification of methylated DNA. The present invention relates to "a method for amplifying a methylated analysis target region; a method for determining the methylation of an analysis target region; a reagent for use in the determination of methylation; a method for determining the occurrence of cancer; a method for obtaining data for determining the occurrence of cancer; a reagent for use in the determination of the occurrence of cancer; and a marker for use in the determination of the occurrence of cancer".

## Description

### Technical Field

The present invention relates to a method for amplifying methylated DNA, a method for determining of methylation of DNA, and a method for determining cancer.

### Background Art

Methylation of DNA is a base modification by DNA methylase and is a state in which a methyl group is added to the 5-position of a cytosine base or the like of the CpG sequence.

Methylation of DNA plays a pivotal role in normal development and cellular differentiation in higher organisms.

Currently, for example, (1) a method in which DNA is subjected to a bisulfite reaction and then the treated DNA is subjected to a nucleic acid amplification reaction such as PCR (Patent Literature 1), or (2) a method in which DNA is treated with a methylation-sensitive restriction enzyme and a methylation-insensitive restriction enzyme and then the treated DNA is subjected to a nucleic acid amplification reaction such as PCR (Patent Literature 2) has been carried out as a method for amplifying methylated DNA. In addition, in Patent Literature 1 and Patent Literature 2, an amplification product obtained by a method for amplifying methylated DNA is analyzed with a DNA microarray or the like, and determination or analysis of whether or not DNA is methylated is also carried out based on the results thus obtained.

On the other hand, methylation of DNA is also known to be associated with canceration. For example, a method for detecting cancer is carried out based on differences in DNA methylation patterns to be associated with cancer and various diseases (Non-Patent Literatures 1 and 2), or DNA methylation rates (Patent Literature 3).

### Citation List

### Patent Literature

Patent Literature 1: WO2013/089063A
Patent Literature 2: WO2009/131223A
Patent Literature 3: JP2008-283947A

### Non-Patent Literature

Non-Patent Literature 1: Yu J et al. Cell Res 13: 319-33, 2003
Non-Patent Literature 2: Kanai Y et al. Hepatology 29: 703-9, 1999

### Summary of Invention

### Technical Problem

However, the method for amplifying methylated DNA in Patent Literature 1 requires a step of subjecting DNA to a bisulfite reaction. In addition, the method for amplifying methylated DNA in Patent Literature 2 requires a step of ligating an adapter to DNA treated with a methylation-insensitive restriction enzyme and then removing the adapter. Therefore, any of these methods is complicated and requires a lot of time.

Accordingly, it has been desired to develop a method capable of conveniently carrying out amplification of methylated DNA, furthermore, determination of methylation of DNA and determination of cancer using the amplification of methylated DNA.

That is, an object of the present invention is to provide a method capable of conveniently carrying out amplification of methylated DNA, and determination of methylation of DNA and determination of cancer using the amplification of methylated DNA.

### Solution to Problem

The present invention has been made for the purpose of achieving the foregoing object and contains the following configuration.
[1] A method for amplifying a methylated analysis target region in double-stranded DNA, comprising the following steps 1 and 2:
   (1) a step 1 of treating double-stranded DNA containing an analysis target region with S1 nuclease and a methylation-sensitive restriction enzyme; and
   (2) a step 2 of amplifying the analysis target region of the double-stranded DNA treated in the step 1.
[2] The amplification method according to [1], in which, in the step 1, treatment with S1 nuclease is carried out and then treatment with a methylation-sensitive restriction enzyme is carried out.
[3] The amplification method according to [1], in which, in the step 1, treatment with a methylation-sensitive restriction enzyme is carried out and then treatment with S1 nuclease is carried out.
[4] The amplification method according to any one of [1] to [3], further comprising a fragmentation step of treating double-stranded DNA with a restriction enzyme whose recognition sequence is not present in the analysis target region, prior to the step 1.
[5] A method for determining the methylation of an analysis target region in double-stranded DNA, comprising the following steps 1 to 4:
   (1) a step 1 of treating double-stranded DNA containing the analysis target region with S1 nuclease and a methylation-sensitive restriction enzyme;
   (2) a step 2 of amplifying the analysis target region of the double-stranded DNA treated in the step 1;
   (3) a step 3 of confirming the presence or absence of an amplification product obtained in the step 2; and
   (4) a step 4 of determining whether or not the analysis target region is methylated based on the results of the step 3.
[6] The methylation determination method according to [5], in which, in the step 1, treatment with S1 nuclease is carried out and then treatment with a methylation-sensitive restriction enzyme is carried out.
[7] The methylation determination method according to [5], in which, in the step 1, treatment with a methylation-sensitive restriction enzyme is carried out and then treatment with S1 nuclease is carried out.
[8] The methylation determination method according to any one of [5] to [7], further comprising a fragmentation step of treating double-stranded DNA with a restriction enzyme whose recognition sequence is not present in the analysis target region, prior to the step 1.
[9] A reagent for determining methylation of DNA, comprising S1 nuclease and a methylation-sensitive restriction enzyme.
[10] A method for determining cancer, comprising the following steps 1 to 4:
   (1) a step 1 of treating double-stranded DNA containing an analysis target region with S1 nuclease and a methylation-sensitive restriction enzyme;
   (2) a step 2 of amplifying the analysis target region of the double-stranded DNA treated in the step 1;
   (3) a step 3 of confirming the presence or absence of an amplification product obtained in the step 2; and
   (4) a step 4 of determining cancer based on the results of the step 3.
[11] The cancer determination method according to [10], in which, in the step 1, treatment with S1 nuclease is carried out and then treatment with a methylation-sensitive restriction enzyme is carried out.
[12] The cancer determination method according to [10], in which, in the step 1, treatment with a methylation-sensitive restriction enzyme is carried out and then treatment with S1 nuclease is carried out.
[13] The cancer determination method according to any one of [10] to [12], further comprising a fragmentation step of treating DNA with a restriction enzyme whose recognition sequence is not present in an analysis target region, prior to the step 1.
[14] The cancer determination method according to any one of [10] to [13], in which the analysis target region is a base sequence containing a base sequence represented by SEQ ID NO: 1, SEQ ID NO: 4, or SEQ ID NO: 11.
[15] A method for obtaining data for determining cancer, comprising the following steps 1 to 3:
   (1) a step 1 of treating double-stranded DNA containing an analysis target region with S1 nuclease and a methylation-sensitive restriction enzyme;
   (2) a step 2 of amplifying the analysis target region of the double-stranded DNA treated in the step 1; and
   (3) a step 3 of confirming the presence or absence of an amplification product obtained in the step 2.
[16] A reagent for determining cancer, comprising S1 nuclease and a methylation-sensitive restriction enzyme.
[17] A marker for determining cancer, obtained by carrying out the following steps 1 and 2:
   (1) a step 1 of treating double-stranded DNA containing an analysis target region with S1 nuclease and a methylation-sensitive restriction enzyme; and
   (2) a step 2 of amplifying the analysis target region of the double-stranded DNA treated in the step 1.

   The present invention further contains the following configurations.
[18] A method for analyzing (determining) methylation of an analysis target region, comprising the following steps 1 to 5:
   (1) a step 1 of treating DNA containing the analysis target region with S1 nuclease;
   (2) a step 2 of treating the double-stranded DNA treated in the step 1 with a methylation-sensitive restriction enzyme;
   (3) a step 3 of amplifying the analysis target region of the double-stranded DNA treated in the step 2;
   (4) a step 4 of confirming the presence or absence of an amplification product obtained in the step 3; and
   (5) a step 5 of determining whether or not the analysis target region is methylated based on the results of the step 4.
[19] The analysis (determination) method according to [18], in which the amplification in the step 3 is carried out by PCR.
[20] The analysis (determination) method according to [18] or [19], in which confirmation of the amplification product in the step 4 is carried out by electrophoresis.
[21] The analysis (determination) method according to any one of [18] to [20], further comprising a purification step of purifying the DNA treated in the step 1, following the step 1.
[22] The analysis (determination) method according to [21], in which the purification in the purification step is carried out by a column purification method.
[23] The analysis (determination) method according to [22], further comprising a fragmentation step of treating DNA with a restriction enzyme whose recognition sequence is not present in the analysis target region, prior to the purification step.
[24] A reagent for analyzing (determining) methylation of DNA, comprising S1 nuclease and a methylation-sensitive restriction enzyme.
[25] A method for determining cancer, comprising the following steps 1 to 5:
   (1) a step 1 of treating DNA containing an analysis target region with S1 nuclease;
   (2) a step 2 of treating the double-stranded DNA treated in the step 1 with a methylation-sensitive restriction enzyme;
   (3) a step 3 of amplifying the analysis target region of the double-stranded DNA treated in the step 2;
   (4) a step 4 of confirming the presence or absence of an amplification product obtained in the step 3; and
   (5) a step 5 of determining cancer based on the results of the step 4.
[26] The determination method according to [25], in which the analysis target region is a promoter region.
[27] The determination method according to [25] or [26], in which the methylation-sensitive restriction enzyme in the step 2 is HapII, HpaII, or SacII.
[28] The determination method according to any one of [25] to [27], in which the nucleic acid to be amplified in the step 3 is a continuous base sequence containing the base sequence represented by SEQ ID NO: 1.
[29] The determination method according to any one of [25] to [28], in which the amplification in the step 3 is carried out by PCR.
[30] The determination method according to [29], in which the PCR is carried out using a forward primer consisting of the base sequence represented by SEQ ID NO: 2 and a reverse primer represented by SEQ ID NO: 3.
[31] The determination method according to any one of [25] to [30], in which the confirmation of the presence or absence of the amplification product in the step 4 is carried out by electrophoresis.
[32] The determination method according to any one of [25] to [31], further comprising a purification step of purifying the DNA treated in the step 1, following the step 1.
[33] The determination method according to [32], in which the purification in the purification step is carried out by a column purification method.
[34] The determination method according to [33], further comprising a fragmentation step of treating DNA with a restriction enzyme whose recognition sequence is not present in the analysis target region, prior to the purification step.
[35] A method for obtaining data for determining cancer, comprising the following steps 1 to 4:
   (1) a step 1 of treating DNA containing an analysis target region with S1 nuclease;
   (2) a step 2 of treating the double-stranded DNA treated in the step 1 with a methylation-sensitive restriction enzyme;
   (3) a step 3 of amplifying the analysis target region of the double-stranded DNA treated in the step 2; and
   (4) a step 4 of confirming the presence or absence of an amplification product obtained in the step 3.
[36] A reagent for determining cancer, comprising S1 nuclease and a methylation-sensitive restriction enzyme.
[37] A marker for determining cancer, obtained by carrying out the following steps 1 to 3:
   (1) a step 1 of treating DNA containing an analysis target region with S1 nuclease;
   (2) a step 2 of treating the double-stranded DNA treated in the step 1 with a methylation-sensitive restriction enzyme; and
   (3) a step 3 of amplifying the analysis target region of the double-stranded DNA treated in the step 2.

### Advantageous Effects of Invention

According to the amplification method, the methylation determination method, the method for determining cancer, and the method for obtaining data for determining cancer of the present invention, amplification of methylated DNA, determination of methylation of DNA, determination of cancer, and acquisition of data for determining cancer can be conveniently carried out in a short period of time.

Further, according to the amplification method, the methylation determination method, the method for determining cancer, and the method for obtaining data for determining cancer of the present invention, non-specific amplification products during the amplification reaction such as PCR can be eliminated, so that amplification of methylated DNA, determination of methylation of DNA, determination of cancer, and acquisition of data for determining of cancer can also be carried out with high accuracy.

### (Brief Description of Drawings)

Fig. 1 is a view showing a flow chart of an amplification method of the present invention divided into various patterns.
Fig. 2 is a view showing a flow chart of a methylation determination method and a method for determining cancer of the present invention divided into various patterns.
Fig. 3 is a view showing a confirmation region of a methylation state in Experimental Example 1 and a sequence recognized by HapII and HpaII [boxed three sequences containing a CpG sequence (CCGG), in a region (267 base pairs) consisting of a base sequence represented by SEQ ID NO: 1 in a human-derived FOXB2 gene promoter region].
Fig. 4 is a view showing whether cytosines in three sequences containing a CpG sequence (CCGG), which are confirmation regions of the methylation state, are methylated cytosines or unmethylated cytosines, for the sequencing results of base sequences of various normal cells and various cancer cells in Experimental Example 1.
Fig. 5 is a view showing the results of electrophoresing a PCR amplification product of a FOXB2 gene using normal cells (hiPS) in Example 1 and Comparative Examples 1 to 3.
Fig. 6 is a view showing the results of electrophoresing the PCR amplification product of a FOXB2 gene using various normal cells in Example 2.
Fig. 7 is a view showing the results of electrophoresing the PCR amplification product of a FOXB2 gene using various cancer cells in Example 2.
Fig. 8 is a view showing the results of electrophoresing the PCR amplification product of a FOXB2 gene using cancer cells derived from a breast cancer patient in Example 3.
Fig. 9 is a view showing the results of electrophoresing the PCR amplification product of a FOXB2 gene using dog-derived cancer cells in Example 4.
Fig. 10 is a view showing a sequence recognized by HpaII and HapII [boxed three sequences containing a CpG sequence (CCGG), in a region (149 base pairs) consisting of a base sequence represented by SEQ ID NO: 11 in the human-derived FOXB2 gene promoter region.
Fig. 11 is a view showing the results of amplifying a specific region of a GAPDH gene by a digital PCR method using plasma derived from a liver cancer patient and a lung cancer patient in Example 5.
Fig. 12 is a view showing the results of amplifying a specific region of a FOXB2 gene by the digital PCR method using plasma derived from a liver cancer patient and a lung cancer patient in Example 5.
Figs. 13A and 13B are views showing the results of simultaneous amplification and detection of specific regions of a FOXB2 gene and a GAPDH gene by the digital PCR method using plasma derived from a normal subject in Example 6.
Figs. 14A and 14B are views showing the results of simultaneous amplification and detection of specific regions of a FOXB2 gene and a GAPDH gene by the digital PCR method using plasma derived from a breast cancer patient in Example 7.
Figs. 15A and 15B are views showing the results of simultaneous amplification and detection of specific regions of a FOXB2 gene and a GAPDH gene by the digital PCR method using plasma derived from a colon cancer patient in Example 7.
Figs. 16A and 16B are views showing the results of simultaneous amplification and detection of specific regions of a FOXB2 gene and a GAPDH gene by the digital PCR method using plasma derived from a pancreatic cancer patient in Example 7.
Figs. 17A and 17B are views showing the results of simultaneous amplification and detection of specific regions of a FOXB2 gene and a GAPDH gene by the digital PCR method using plasma derived from a gastric cancer patient in Example 7.
Figs. 18A and 18B are views showing the results of simultaneous amplification and detection of specific regions of a FOXB2 gene and a GAPDH gene by the digital PCR method using plasma derived from a lung cancer patient after administration of an anticancer agent in Example 7.

### Description of Embodiments

### Amplification method according to embodiment of present invention

The amplification method according to the embodiment of the present invention contains (1) a step 1 of treating double-stranded DNA containing an analysis target region with S1 nuclease and a methylation-sensitive restriction enzyme, and (2) a step 2 of amplifying the analysis target region of the double-stranded DNA treated in the step 1.

### Double-stranded DNA and extraction of the DNA

The double-stranded DNA according to the present invention may be any double-stranded DNA containing a CpG sequence (-CG-).

The double-stranded DNA according to the present invention may be extracted from a sample containing the DNA, and the extraction method may be carried out based on a DNA extraction method known per se. Specific examples of the extraction method of the DNA include a method of mixing a sample with a treatment liquid containing a surfactant (sodium cholate, sodium dodecyl sulfate, or the like), and subjecting the mixed liquid obtained to a physical treatment (stirring, homogenization, ultrasonic grinding, or the like) so that the DNA contained in the sample is liberated into the mixed liquid to thereby extract DNA; a method of extracting DNA from a sample using phenol-chloroform; and a column extraction method. Among them, a method of extracting DNA from a sample using phenol-chloroform or a column extraction method is more preferable.

The method of extracting DNA from a sample using phenol-chloroform is a method of extracting DNA excluding a protein present in a sample, on the basis of the property of phenol which denatures the protein contained in the sample and the property of chloroform which promotes such an action. A specific method may be carried out based on a method known per se.

In addition, the DNA extraction method may be carried out using a commercially available kit [for example, Phase Lock Gel (manufactured by QTB Corp.)].

On the other hand, the column extraction method is a method in which a sample is added to a cylindrical container such as a column comprising a membrane or the like therein, and DNA is extracted from the sample, utilizing a difference in substance size, adsorption power, charge, hydrophobicity, or the like. A specific method may be carried out based on a method known per se.

In addition, the DNA extraction method may be carried out using a commercially available kit [for example, QuickGene SP kit DNA tissue (manufactured by Kurabo Industries Ltd.), MagMAX™ Cell-Free DNA Isolation Kit (manufactured by Thermo Fisher Scientific Inc.), or NucleoSpin™ Plasma XS (manufactured by Macherey-Nagel Inc.)].

Specific examples of the sample include a body fluid such as whole blood, serum, plasma, or urine; a tissue; and a cell. In the case where such a body fluid such as whole blood, serum, plasma, or urine; a tissue, a cell, or the like is used as a sample, the amplification product obtained by the amplification method according to the embodiment of the present invention can be used for the determination of methylation of DNA, or for determination or/and diagnosis of disease associated with methylation of DNA.

Examples of the double-stranded DNA according to the present invention in the case of using the sample include double-stranded DNA derived from circulating cancer (tumor) cells (CTCs) present in body fluids such as serum, plasma, and urine; double-stranded DNA [cell free DNA (cfDNA)] derived from the death of cancer (tumor) cells, blood cells such as white blood cells, and cells or the like derived from individual tissues; double-stranded DNA derived from exosome; and double-stranded DNA derived from cells present in tissues, cells, whole blood, urine, and the like.

### Analysis target region

The analysis target region according to the present invention is a continuous specific double-stranded DNA part containing a CpG sequence present in the double-stranded DNA according to the present invention, and may be part or all of the double-stranded DNA according to the present invention.

Among such analysis target regions, the analysis target region which is amplified in the amplification method according to the embodiment of the present invention is a continuous specific double-stranded DNA part containing a CpG sequence in which cytosine is methylated.

The term "methylation" as used herein refers to a base modification by a DNA methylase, meaning a state in which a methyl group is added to the 5-position of a cytosine base or the like of a CpG sequence which is a two-base sequence in which guanine appears next to cytosine.

The size (number of base pairs) of the analysis target region according to the present invention is usually 50 to 1000 base pairs, preferably 50 to 700 base pairs, and more preferably 50 to 500 base pairs.

A specific example of the analysis target region according to the present invention may be DNA of a specific region within the promoter region of any selected appropriate gene, and is more preferably DNA of a specific region within the promoter region of a FOXB2 gene; still more preferably DNA of a specific region within the promoter region of a FOXB2 gene derived from human or DNA of a specific region within the promoter region of a FOXB2 gene derived from a dog; and particularly preferably a region (267 base pairs) consisting of the base sequence represented by SEQ ID NO: 1 within the promoter region of the FOXB2 gene derived from human, a region (149 base pairs) consisting of the base sequence represented by SEQ ID NO: 11 within the promoter region of the FOXB2 gene derived from human, or a region (433 base pairs) consisting of the base sequence represented by SEQ ID NO: 4 within the promoter region of the FOXB2 gene derived from a dog.

### Step 1

The step 1 according to the present invention is a step of treating double-stranded DNA containing an analysis target region with S1 nuclease and a methylation-sensitive restriction enzyme.

The S1 nuclease in the step 1 according to the present invention is a type of single strand-specific nuclease and is an enzyme that degrades a single-stranded region of single-stranded DNA and double-stranded DNA.

The concentration (number of units) of S1 nuclease in the step 1 according to the present invention is usually 1 to 200 units/µL, preferably 5 to 190 units/µL, and more preferably 20 to 180 units/µL with respect to 1 to 200 ng of the target DNA.

The treatment with S1 nuclease in the step 1 according to the present invention is carried out usually at 10°C to 37°C and preferably at 15°C to 25°C, usually for 5 to 30 minutes and preferably for 10 to 20 minutes.

In addition, the treatment with S1 nuclease in the step 1 according to the present invention is preferably carried out under the conditions of pH 4 to 5. As a buffer solution used here, for example, a sodium acetate buffer solution or the like may be used.

The methylation-sensitive restriction enzyme in the step 1 according to the present invention is a restriction enzyme whose cleavage activity is affected depending on whether or not cytosine in a CpG sequence in the recognition sequence is methylated. That is, the methylation-sensitive restriction enzyme is a restriction enzyme which is incapable of cleaving a recognition sequence in the case where the recognition sequence has a CpG sequence containing a methylated cytosine base, but is capable of cleaving a recognition sequence in the case where the recognition sequence does not have a CpG sequence containing a methylated cytosine base.

The methylation-sensitive restriction enzyme in the step 1 according to the present invention may be any methylation-sensitive restriction enzyme as long as the base sequence to be recognized is present in the analysis target region. That is, the methylation-sensitive restriction enzyme may be appropriately selected depending on the analysis target region.

Specific examples of the methylation-sensitive restriction enzyme in the step 1 according to the present invention include AatII, AccI, AccII, AfaI, Aor13HI, Aor51HI, ApaI, ApaLI, BglI, BmgT120I, BspT104I, BssHII, Cfr10I, ClaI, CpoI, EaeI, Eco52I, HaeII, HapII, HpaII, HhaI, MluI, NaeI, NheI, NotI, NruI, NsbI, PmaCI, Psp1406I, PvuI, SacII, SalI, Sau3AI, SmaI, SnaBI, and VpaK11BI. Among them, in the case where a region consisting of the base sequence represented by SEQ ID NO: 1 within the promoter region of the human-derived FOXB2 gene is set as the analysis target region, it is preferable to use HapII, HpaII, or SacII and it is more preferable to use HpaII or HapII. In addition, in the case where a region consisting of the base sequence represented by SEQ ID NO: 11 within the promoter region of the human-derived FOXB2 gene or a region consisting of the base sequence represented by SEQ ID NO: 4 within the promoter region of the FOXB2 gene derived from a dog is set as the analysis target region, it is preferable to use HapII or HpaII.

The concentration (number of units) of the methylation-sensitive restriction enzyme in the step 1 according to the present invention is usually 1 to 50 units/µL, preferably 5 to 50 units/µL, and more preferably 10 to 50 units/µL with respect to 1 to 200 ng of the target DNA.

The treatment with the methylation-sensitive restriction enzyme in the step 1 according to the present invention may be carried out usually at 20°C to 50°C and preferably at 35°C to 45°C, usually for 60 to 150 minutes and preferably for 60 to 120 minutes.

In addition, the treatment with the methylation-sensitive restriction enzyme in the step 1 according to the present invention is preferably carried out under the conditions of pH 6 to 8. As a buffer solution used here, for example, a Tris buffer solution or the like may be used.

In the step 1 according to the present invention, (i) the treatment with S1 nuclease and the treatment with a methylation-sensitive restriction enzyme may be carried out at the same time, (ii) the treatment with S1 nuclease may be followed by the treatment with a methylation-sensitive restriction enzyme, or (iii) the treatment with a methylation-sensitive restriction enzyme may be followed by the treatment with S1 nuclease; but since the treatment with S1 nuclease and the treatment with a methylation-sensitive restriction enzyme have different preferred pH values, (ii) or (iii) is more preferable and (ii) is still more preferable.

Further, it is preferable to purify the DNA containing the analysis target region after the treatment with S1 nuclease or/and the treatment with a methylation-sensitive restriction enzyme in the step 1 according to the present invention. The details of the purification will be described in detail in the section of **"Purification step".**

In addition, prior to the step 1 according to the present invention, it is preferable to carry out the treatment with a restriction enzyme whose recognition sequence is not present in the analysis target region. The details of the restriction enzyme will be described in detail in the section of **"Fragmentation step of treating double-stranded DNA with restriction enzyme whose recognition sequence is not present in the analysis target region".**

Thus, single-stranded DNA generated by artificial manipulation and endogenous phenomena (loop region in DNA-DNA or DNA-RNA hybrid) can be specifically cleaved by carrying out the treatment with S1 nuclease in the step 1 according to the present invention. As a result, generation of non-specific amplification products is suppressed in the step 2 according to the present invention (step of amplifying the analysis target region of the double-stranded DNA treated in the step 1 according to the present invention).

In addition, the analysis target region of the methylated double-stranded DNA can be specifically amplified since the analysis target region of the methylated double-stranded DNA is not cleaved, and the analysis target region of the unmethylated double-stranded DNA is cleaved, by carrying out the treatment with a methylation-sensitive restriction enzyme in the step 1 according to the present invention.

Therefore, in the step 2 according to the present invention, the analysis target region of the methylated double-stranded DNA can be amplified with specificity and high sensitivity by treating the DNA containing the analysis target region in the step 1 according to the present invention.

### Step 2

The step 2 according to the present invention is a step of amplifying the analysis target region of the double-stranded DNA treated in the step 1 according to the present invention.

The amplification product in the step 2 according to the present invention may be the analysis target region itself or may contain a base sequence other than the analysis target region (for example, a primer). The size (number of base pairs) of the amplification product is usually 50 to 1100 base pairs, preferably 50 to 800 base pairs, and more preferably 50 to 600 base pairs.

A specific example of the amplification product in the step 2 according to the present invention may be DNA of a specific region within the promoter region of any selected appropriate gene, and is more preferably DNA of a specific region within the promoter region of a FOXB2 gene; still more preferably DNA of a specific region within the promoter region of a FOXB2 gene derived from human or DNA of a specific region within the promoter region of a FOXB2 gene derived from a dog; and particularly preferably a region (267 base pairs) consisting of the base sequence represented by SEQ ID NO: 1 within the promoter region of the FOXB2 gene derived from human, a region (149 base pairs) consisting of the base sequence represented by SEQ ID NO: 11 within the promoter region of the FOXB2 gene derived from human, or a region (433 base pairs) consisting of the base sequence represented by SEQ ID NO: 4 within the promoter region of the FOXB2 gene derived from a dog.

The method of amplifying the analysis target region in the step 2 according to the present invention is not particularly limited, and may be carried out based on a method known per se. Specific examples of such an amplification method known per se include a polymerase chain reaction (PCR) method and a loop-mediated isothermal amplification (LAMP) method, among which the PCR method is more preferable.

The PCR method may be carried out by a known method using a primer, a nucleic acid synthesis enzyme, a nucleic acid synthesis substrate, a buffer solution, if necessary, a probe,, and the like. Specifically, the PCR method may be carried out based on the method described in, for example, Nucleic Acids Research, 1991, Vol. 19, p. 3749; BioTechniques, 1994, Vol. 16, pp. 1134 to 1137; and "PCR Experiment Protocol Selectable by Objective, 2011, pp. 56 to 73, 120 to 131".

The PCR method is preferably a digital PCR method, in the case where a specific region of DNA derived from CTC, a specific region of cfDNA, or a specific region of DNA derived from exosome, which is present in a body fluid such as serum, plasma, or urine, is set as an analysis target region.

In the digital PCR method, a PCR reaction is carried out by dividing a PCR reaction solution into a plurality of tiny compartments so that the DNA fragment becomes one molecule, and the target DNA is detected and quantified based on the number of the tiny compartments in which the amplification product is detected. The digital PCR method may be carried out based on, for example, the method described in JP2014-506465A and the method described in Proc. Natl. Acad. Sci. USA Vol. 96, pp. 9236 to 9241, August 1999 Genetics.

Specifically, for example, a reaction solution for digital PCR containing a DNA-containing solution as a template, a primer pair consisting of a forward primer and a reverse primer, a probe, a nucleic acid synthesis substrate, a nucleic acid synthesis enzyme, and the like is prepared, and the reaction solution is divided into a number of droplets (10,000 to 30,000 droplets per sample) by a droplet producing device [for example, QX200 Droplet Generator (manufactured by Bio-Rad Laboratories Ltd.) or Automated Droplet Generator (manufactured by Bio-Rad Laboratories Ltd.)]. Thereafter, the PCR reaction may be carried out with a digital PCR device [for example, Droplet Digital PCR (manufactured by Bio-Rad Laboratories Ltd.) or QuantStudio 3D Digital PCR System (manufactured by Thermo Fisher Scientific Co., Ltd.)].

The primer pair (hereinafter, sometimes referred to simply as a primer pair according to the present invention) consisting of a forward primer and a reverse primer in the PCR method (hereinafter, sometimes referred to simply as a primer according to the present invention) may be any primer pair as long as it is designed to amplify the analysis target region according to the present invention.

The size (number of bases) of the primers constituting the primer pair according to the present invention is usually 10 to 50 bases, preferably 10 to 35 bases, more preferably 15 to 35 bases, and particularly preferably 20 to 30 bases.

Specifically, for the primer pair according to the present invention, for example, in the case where a region consisting of the base sequence represented by SEQ ID NO: 1 within the promoter region of the human-derived FOXB2 gene is set as the analysis target region, the forward primer is preferably one consisting of the base sequence represented by SEQ ID NO: 2, and the reverse primer is preferably one consisting of the base sequence represented by SEQ ID NO: 3. In the case where a region consisting of the base sequence represented by SEQ ID NO: 11 within the promoter region of the human-derived FOXB2 gene is set as the analysis target region, the forward primer is preferably one consisting of the base sequence represented by SEQ ID NO: 12, and the reverse primer is preferably one consisting of the base sequence represented by SEQ ID NO: 13. In addition, in the case where a region consisting of the base sequence represented by SEQ ID NO: 4 within the promoter region of the FOXB2 gene derived from a dog is set as the analysis target region, the forward primer is preferably one consisting of the base sequence represented by SEQ ID NO: 5, and the reverse primer is preferably one consisting of the base sequence represented by SEQ ID NO: 6.

Hereinafter, the base sequences and designations of individual primers constituting the primer pair, and regions to be amplified by the primer pair will be shown.

**Table 1**

| **Primer** | | **Designation** | **SEQ ID NO:** | **Region to be amplified** |
|---|---|---|---|---|
| Forward | caaggtcgga gacaccaggc aattc | h-F-fox | 2 | SEQ ID NO: 1 (267 base pairs) |
| Reverse | ccatggcggt cagcgagatg taag | h-R-fox | 3 | |
| Forward | ctggaggggc cgagtgggaa ttag | hcf-F-fox | 12 | SEQ ID NO: 11 (149 base pairs) |
| Reverse | gtttttggtc gctgtacgag ctcttc | hcf-R-fox | 13 | |
| Forward | gagctccctt ctcctgacct eg | d-F-fox | 5 | SEQ ID NO: 4 (433 base pairs) |
| Reverse | ctctccggcc agctccgctc tc | d-R-fox | 6 | |

In addition, for the primer pair according to the present invention, it is preferable to use the primer pair according to the present invention as it is, but the primer pair may be one consisting of primers in which either or both of the forward primer and the reverse primer are labeled with a labeling substance.

The method of labeling the primer according to the present invention with a labeling substance is not particularly limited, and may be carried out based on a method known per se.

Examples of the labeling substance, which is used for labeling the primer according to the present invention with a labeling substance, include known labeling substances such as a fluorescent substance, a radioisotope, an enzyme, and a luminescent substance, among which a fluorescent substance is particularly preferable.

Examples of the fluorescent substance include TAMRA™ (manufactured by Sigma-Aldrich Co. LLC), Alexa555, Alexa647 (manufactured by Invitrogen Corporation), cyanine dyes Cy3 and Cy5 (manufactured by Amersham Biosciences, Inc.), and fluorescein.

Examples of the radioisotope include ³²P, ³³P, and ³⁵S.

Examples of the enzyme include alkaline phosphatase and horseradish peroxidase.

The luminescent substance may be, for example, a chemiluminescent reagent containing Acridinium Easter.

The method of labeling the primer according to the present invention with a fluorescent substance may be, for example, a method in which a fluorescein-labeled nucleotide is incorporated into a primer based on a method known per se. In addition, nucleotides can also be labeled with a fluorescent substance by a method of substituting a nucleotide having a linker arm in an oligonucleotide of a sequence (see Nucleic Acids Res., 1986, Vol. 14, p. 6115). For example, there is a method in which uridine having a linker arm at the 5-position is chemically synthesized from deoxyuridine by the synthesis method disclosed in JP1985-500717A (JP-S60-500717A), an oligonucleotide containing the deoxyuridine is synthesized, and then a fluorescent substance is introduced into the oligonucleotide chain (JP1985-500717A (JP-S60-500717A)).

As the method of labeling the primer according to the present invention with a radioisotope, there are a method of incorporating a radioisotope-labeled nucleotide to label a primer at the time of synthesizing the primer, and a method of synthesizing a primer and then labeling the primer with a radioisotope. Specific examples of the primer labeling method include the commonly used random primer method, nick translation, 5' end labeling method with T4 polynucleotide kinase, and 3' end labeling method using terminal deoxynucleotidyl transferase, and the like.

The method for labeling the primer according to the present invention with an enzyme may be, for example, a direct labeling method such as direct covalent bonding of an enzyme molecule such as alkaline phosphatase or horseradish peroxidase to a primer to be labeled.

The method of labeling the primer according to the present invention with a luminescent substance may be, for example, a method of luminescently labeling a nucleotide based on a method known per se.

In addition, the labeling substance may be bound to the primer according to the present invention according to the "detection system utilizing a biotin-avidin reaction". In that case, the labeling may be carried out based on a method known per se.

A probe in the PCR method (hereinafter, sometimes referred to simply as a probe according to the present invention) may be any probe as long as it is a probe designed so as to hybridize to a region to be amplified in the case where the amplification method according to the embodiment of the present invention is carried out with the primer pair according to the present invention, the 5' end thereof is labeled with a reporter fluorescent dye, and the 3' end thereof is labeled with a quencher fluorescent dye.

The size (number of bases) of the probe according to the present invention is usually 10 to 50 bases, preferably 15 to 40 bases, and more preferably 20 to 30 bases.

Specifically, for example, in the case where a region consisting of the base sequence represented by SEQ ID NO: 11 within the promoter region of the human-derived FOXB2 gene is set as the analysis target region, the probe according to the present invention is preferably a probe consisting of the base sequence represented by SEQ ID NO: 14.

The base sequence and designation of the probe are shown below.

**Table 2**

| **Probe** | **Designation** | **SEQ ID NO:** |
|---|---|---|
| cacagaatct ctccgcactc cgttc | hcf-Probe-fox | 14 |

The method of labeling the 5' end of the probe according to the present invention with a reporter fluorescent dye and labeling the 3' end of the probe with a quencher fluorescent dye is the same as the method of labeling the primer pair according to the present invention with a labeling substance.

Examples of the reporter fluorescent dye, which is used for labeling the probe according to the present invention with a reporter fluorescent dye and a quencher fluorescent dye, include FAM, HEX, and VIC, and examples of the quencher fluorescent dye include TAMRA, BHQ1, and BHQ2.

Incidentally, in the case where a probe consisting of the base sequence represented by SEQ ID NO: 14 is used as the probe according to the present invention, FAM is preferable as the reporter fluorescent dye, and BHQ1 is preferable as the quencher fluorescent dye.

In addition, examples of the nucleic acid synthesis enzyme in the PCR method include Taq DNA polymerase and KOD DNA polymerase. Examples of the nucleic acid synthesis substrate include dNTPs (dATP, dCTP, dGTP, and dGTTP). Examples of the buffer solution include a Tris buffer solution and a phosphate buffer solution, but any buffer solution commonly used in the art may be used.

Further, the reaction solution for PCR may contain a salt such as MgCl₂, KCl, or (NH₄)₂SO₄, a surfactant such as polyethylene glycol, Triton (manufactured by Union Carbide Corporation), Nohidet (manufactured by Shell Chemicals Co., Ltd.), or CHAPS (manufactured by Dojindo Laboratories Co., Ltd.), a preservative such as ProClin 300 (manufactured by Sigma-Aldrich Co. LLC), a methylation-sensitive restriction enzyme such as HapII, HpaII, or SacII, and the like.

Thus, the methylated analysis target region remaining without being cleaved by the methylation-sensitive restriction enzyme in the step 1 according to the present invention can be specifically amplified by carrying out the step 2 according to the present invention.

### Purification step

The purification step according to the present invention is a step of purifying DNA.

As a result, it is preferable to carry out the purification step because impurities can be removed before the subsequent treatment or step.

In the case of carrying out the purification step according to the present invention, the purification step may be carried out after the step 1 according to the present invention and before the step 2 according to the present invention, or/and after **"Fragmentation step of treating double-stranded DNA with restriction enzyme whose recognition sequence is not present in analysis target region"** to be described later and before the step 1 according to the present invention.

Further, the following cases can be mentioned as a case where the purification step is carried out after the step 1 according to the present invention and before the step 2 according to the present invention.
(i) In the case where the treatment with S1 nuclease and the treatment with a methylation-sensitive restriction enzyme are carried out at the same time in the step 1 according to the present invention, the purification step may be carried out after these treatments and before the step 2 according to the present invention.
(ii) In the case where the treatment with S 1 nuclease is followed by the treatment with a methylation-sensitive restriction enzyme in the step 1 according to the present invention, the purification step may be carried out after the treatment with S1 nuclease or/and after the treatment with a methylation-sensitive restriction enzyme, and before the step 2 according to the present invention, and it is preferable to carry out the purification step after at least the treatment with S1 nuclease (and before the treatment with a methylation-sensitive restriction enzyme).
(iii) In the case where the treatment with a methylation-sensitive restriction enzyme is followed by the treatment with S 1 nuclease in the step 1 according to the present invention, the purification step may be carried out after the treatment with a methylation-sensitive restriction enzyme or/and after the treatment with S1 nuclease, and before the step 2 according to the present invention, and it is preferable to carry out the purification step after the treatment with a methylation-sensitive restriction enzyme and after the treatment with S 1 nuclease, and before the step 2 according to the present invention, respectively.

The preferred pH is different between the treatment with S1 nuclease in the step 1 according to the present invention and the treatment with a methylation-sensitive restriction enzyme in the step 1 according to the present invention and the step 2 according to the present invention. Therefore, in the case of (ii), following the treatment with S1 nuclease, the purification step is subsequently carried out to remove impurities and adjust the pH, so that the reaction of the methylation-sensitive restriction enzyme in the step 1 according to the present invention can be carried out efficiently.

In addition, in the case of (iii), following the treatment with a methylation-sensitive restriction enzyme and the treatment with S1 nuclease, the purification step is subsequently carried out to remove impurities and adjust the pH, so that the step 2 according to the present invention can be carried out efficiently.

The method of carrying out the purification step according to the present invention is not particularly limited as long as it is a per se known purification method commonly carried out in the art. Specific examples of the purification method include an alcohol precipitation method and a column purification method, among which a column purification method is more preferable from the viewpoint of being applicable to high throughput.

The alcohol precipitation method is a method of purifying DNA by adding an alcohol to a DNA-containing solution and precipitating the DNA by utilizing the property of DNA which is a hydrophilic polymer. In the case where the purification is carried out by the alcohol precipitation method, for example, it may be carried out as follows.

That is, 50 to 100 µL of an alcohol and 30 to 100 µL of a buffer solution are added to 10 to 100 µL of the DNA-containing solution, followed by centrifugation at 10,000 to 22,000 ×g for 1 to 30 minutes, and the flow-through solution is recovered, whereby the purified DNA is obtained.

Examples of the alcohol include isopropanol, ethanol, and butanol, among which isopropanol or ethanol is more preferable, and isopropanol is still more preferable.

Examples of the buffer solution include a Tris buffer solution, a phosphate buffer solution, and a glycine buffer solution, among which a Tris buffer solution or a phosphate buffer solution is more preferable, and a Tris buffer solution is still more preferable. Incidentally, the concentration and pH of the buffer solution may be within any range commonly used in the art.

In addition, the purification method may be carried out using a commercially available kit.

The column purification method is a method in which a DNA-containing solution is added to a cylindrical container [for example, ECONOSPIN (manufactured by Gene Design Inc.) or Mobi Spin S-4000 (manufactured by Molecular Biotechnology Co., Ltd.)] such as a column comprising a filler such as silica gel, polyacrylamide gel, or Sephacryl, and the DNA is purified using the difference in affinity between the DNA and the filler. In the case where the purification is carried out by a column purification method, for example, it may be carried out as follows.

That is, 200 to 700 µL of a protein denaturing agent or/and 200 to 700 µL of a buffer solution are added to and mixed with 10 to 150 µL of the DNA-containing solution, and the mixture is transferred to, for example, a column filled with a filler [for example, ECONOSPIN (manufactured by Gene Design Inc.)] and centrifuged at 10000 to 22000 ×g and room temperature for 1 to 3 minutes to remove the solution in the tube. Next, 500 to 700 µL of a buffer solution and 500 to 700 µL of an alcohol are added thereto, followed by centrifugation at 10000 to 22000 ×g and room temperature for 1 to 3 minutes to remove the solution in the tube. Furthermore, centrifugation is carried out at 10000 to 22000 ×g and room temperature for 1 to 10 minutes, the tube is changed to a fresh one, and 20 to 60 µL of buffer solution or sterile water is added, followed by centrifugation at 10000 to 22000 ×g at room temperature for 1 to 3 minutes to recover a flow-through solution, whereby the purified DNA is obtained.

Examples of the protein denaturing agent include guanidine hydrochloride, formamide, and urea, among which guanidine hydrochloride is more preferable.

Examples of the alcohol include ethanol, isopropanol, and butanol, among which ethanol or isopropanol is more preferable, and ethanol is still more preferable.

Examples of the buffer solution include a Tris buffer solution, a phosphate buffer solution, and a glycine buffer solution, among which a Tris buffer solution or a phosphate buffer solution is more preferable, and a Tris buffer solution is still more preferable. Incidentally, the concentration and pH of the buffer solution may be within any range commonly used in the art.

In addition, the purification method may be carried out using a commercially available kit.

### Fragmentation step of treating double-stranded DNA with restriction enzyme whose recognition sequence is not present in the analysis target region

In the amplification method according to the embodiment of the present invention, it is preferable to carry out a fragmentation step in which DNA containing an analysis target region is extracted from a sample and then treated with a restriction enzyme whose recognition sequence is not present in the analysis target region (hereinafter, sometimes referred to simply as a fragmentation step according to the present invention). In particular, it is preferable to carry out the fragmentation step in the case where a specific region of DNA derived from CTC, a specific region of cfDNA, or a specific region of DNA derived from exosome, which is present in a body fluid such as serum, plasma, or urine, is set as an analysis target region. In addition, in the case of carrying out the fragmentation step according to the present invention, the fragmentation step may be carried out before carrying out the step 1 according to the present invention.

The restriction enzyme in the fragmentation step according to the present invention is a restriction enzyme capable of cleaving double-stranded DNA and it may be any one as long as the recognition sequence thereof is not present in the analysis target region, and may be appropriately selected depending on the analysis target region. Furthermore, a plurality of restriction enzymes may be used in the fragmentation step according to the present invention, if necessary.

The expression "whose recognition sequence is not present in the analysis target region" has the same meaning as "the recognition sequence of the restriction enzyme cannot cleave the analysis target region" or "the restriction enzyme does not recognize the base sequence in the analysis target region".

As the restriction enzyme in the fragmentation step according to the present invention, it is preferable to use BamHI or/and HindIII in the case where a region consisting of the base sequence represented by SEQ ID NO: 1 within the promoter region of the human-derived FOXB2 gene, or a region consisting of the base sequence represented by SEQ ID NO: 4 within the promoter region of the dog-derived FOXB2 gene is set as the analysis target region. In addition, it is preferable to use MboI in the case where a region consisting of the base sequence represented by SEQ ID NO: 11 within the promoter region of the human-derived FOXB2 gene is set as the analysis target region.

The concentration (number of units) of the restriction enzyme in the fragmentation step according to the present invention is usually 0.5 to 30 units/µL and preferably 1 to 25 units/µL with respect to 1 to 200 ng of the target DNA. In addition, the concentration (number of units) of the restriction enzyme in the fragmentation step according to the present invention in the case of treatment using a plurality of restriction enzymes is also the same as above and is usually 0.5 to 30 units/µL and preferably 1 to 25 units/µL.

The treatment with a restriction enzyme in the fragmentation step according to the present invention is usually carried out at 20°C to 50°C and preferably at 35°C to 40°C, usually for 60 to 150 minutes and preferably for 60 to 120 minutes.

In addition, the fragmentation step according to the present invention is preferably carried out under the conditions of pH 6 to 8. As a buffer solution used here, for example, a Tris buffer solution or the like may be used.

As described above, the preferred pH in the fragmentation step according to the present invention and the treatment with a methylation-sensitive restriction enzyme in the step 1 according to the present invention is the same. Therefore, in addition to the treatment with a methylation-sensitive restriction enzyme in the step 1 according to the present invention, the treatment with a restriction enzyme in the fragmentation step according to the present invention and the treatment with a methylation-sensitive restriction enzyme may be carried out at the same time.

As described above, DNA outside the analysis target region in the DNA can be fragmented by carrying out the fragmentation step according to the present invention, so that the reaction in the subsequent step can be efficiently carried out.

Incidentally, the purification step may be carried out after the fragmentation step.

### Specific examples of amplification method according to embodiment of present invention

Hereinafter, specific examples of the amplification method according to the embodiment of the present invention will be described separately in the case (A) where a specific region of DNA present in a body fluid such as serum, plasma, or urine is set as an analysis target region, and in the case (B) where a specific region of DNA present in tissue, cell, whole blood, urine, or the like is set as an analysis target region.

### (A) In case where specific region of DNA present in body fluid such as serum, plasma, or urine (for example, region consisting of the base sequence represented by SEQ ID NO: 11) is set as analysis target region

### (1) Extraction of DNA

DNA (for example, cfDNA) is extracted from a sample (for example, plasma), using, for example, a nucleic acid extraction kit [for example, NucleoSpin™ Plasma XS (manufactured by Macherey-Nagel Inc.) or MagMAX™ Cell-Free DNA Isolation Kit (manufactured by Thermo Fisher Scientific Inc.)].

### (2) Fragmentation step according to present invention

5 to 15 µL of a buffer solution (for example, a Tris buffer solution) in the fragmentation step according to the present invention and 0.5 to 5 µL of a restriction enzyme (for example, MboI) in the fragmentation step of 0.5 to 30 units/µL are added to the DNA obtained in the section (1), which is then adjusted to a volume of 50 to 100 µL with sterile water. Incidentally, it is preferable to contain 0.5 to 5 µL of 1 to 50 units/µLmethylation-sensitive restriction enzyme (for example, HapII or HpaII).

Thereafter, the reaction mixture is reacted at 20°C to 50°C for 60 to 150 minutes to obtain a solution treated in the fragmentation step according to the present invention.

Further, if necessary, the solution treated in the fragmentation step according to the present invention may be subjected to the purification step according to the present invention as follows.

That is, 500 to 700 µL of a protein denaturing agent (for example, guanidine hydrochloride) and 500 to 700 µL of a buffer solution (for example, a Tris buffer solution) with a pH of 5 to 8 are added to and mixed with the solution treated in the fragmentation step according to the present invention. Then, the mixture is transferred to, for example, a column filled with a filler [for example, ECONOSPIN (manufactured by Gene Design Inc.)] and centrifuged at 10000 to 22000 ×g and room temperature for 1 to 3 minutes to remove the solution in the tube. Thereafter, 500 to 700 µL of a buffer solution (for example, a Tris buffer solution) with a pH of 5 to 8 and 500 to 700 µL of an alcohol (for example, ethanol) are added thereto, followed by centrifugation at 10000 to 22000 ×g and room temperature for 1 to 5 minutes to remove the solution in the tube. This operation is repeated once more, followed by further centrifugation at 10000 to 22000 ×g and room temperature for 1 to 5 minutes, and the tube is changed to a fresh one, followed by addition of 20 to 60 µL of sterile water and centrifugation at 10000 to 22000 ×g and room temperature for 1 to 3 minutes to recover a flow-through solution.

The above obtained solution or flow-through solution treated in the fragmentation step according to the present invention is subjected to the step 1 according to the present invention.

### (3) Step 1 according to present invention

### (3)-1 Treatment with S1 nuclease

To 40 to 60 µL of the solution or flow-through solution treated in the fragmentation step according to the present invention are added 20 to 50 µL of sterile water, 5 to 15 µL of a buffer solution (for example, a sodium acetate buffer solution) in the step 1 according to the present invention, and 0.5 to 1.5 µL of 1 to 200 units/µL S1 nuclease, followed by reaction at 10°C to 37°C for 10 to 20 minutes to obtain a solution treated in the step 1 according to the present invention (treatment with S1 nuclease).

Further, if necessary, the solution treated in the step 1 according to the present invention (treatment with S1 nuclease) may be subjected to the purification step according to the present invention as follows.

That is, 500 to 700 µL of a protein denaturing agent (for example, guanidine hydrochloride) and 500 to 700 µL of a buffer solution (for example, a Tris buffer solution) with a pH of 5 to 8 are added to and mixed with the solution treated in the step 1 according to the present invention (treatment with S1 nuclease). Then, the mixture is transferred to, for example, a column filled with a filler [for example, ECONOSPIN (manufactured by Gene Design Inc.)] and centrifuged at 10000 to 22000 ×g and room temperature for 1 to 3 minutes to remove the solution in the tube. Thereafter, 500 to 700 µL of a buffer solution (for example, a Tris buffer solution) with a pH of 5 to 8 and 500 to 700 µL of an alcohol (for example, ethanol) are added thereto, followed by centrifugation at 10000 to 22000 ×g and room temperature for 1 to 5 minutes to remove the solution in the tube. This operation is repeated once more, followed by further centrifugation at 10000 to 22000 ×g and room temperature for 1 to 5 minutes, and the tube is changed to a fresh one, followed by addition of 20 to 60 µL of sterile water and centrifugation at 10000 to 22000 ×g and room temperature for 1 to 3 minutes to recover a flow-through solution.

The above obtained solution or flow-through solution treated in the step 1 according to the present invention (treatment with S1 nuclease) is subjected to the step 1 according to the present invention (treatment with a methylation-sensitive restriction enzyme).

### (3)-2 Treatment with methylation-sensitive restriction enzyme

To 40 to 60 µL of the solution or flow-through solution treated in the step 1 according to the present invention (treatment with S1 nuclease) are added 20 to 50 µL of sterile water, 5 to 15 µL of a buffer solution (for example, a Tris buffer solution) in the step 1 according to the present invention, and 1 to 5 µL of 1 to 50 units/µL methylation-sensitive restriction enzyme (for example, HapII or HpaII), followed by reaction at 20°C to 50°C for 60 to 150 minutes to obtain a solution treated in the step 1 according to the present invention (treatment with a methylation-sensitive restriction enzyme).

Further, if necessary, the solution treated in the step 1 according to the present invention (treatment with a methylation-sensitive restriction enzyme) may be subjected to the purification step according to the present invention as follows.

That is, 500 to 700 µL of a protein denaturing agent (for example, guanidine hydrochloride) and 500 to 700 µL of a buffer solution (for example, a Tris buffer solution) with a pH of 5 to 8 are added to and mixed with the solution treated in the step 1 according to the present invention (treatment with a methylation-sensitive restriction enzyme). Then, the mixture is transferred to, for example, a column filled with a filler [for example, ECONOSPIN (manufactured by Gene Design Inc.)] and centrifuged at 10000 to 22000 ×g and room temperature for 1 to 3 minutes to remove the solution in the tube. Thereafter, 500 to 700 µL of a buffer solution (for example, a Tris buffer solution) with a pH of 5 to 8 and 500 to 700 µL of an alcohol (for example, ethanol) are added thereto, followed by centrifugation at 10000 to 22000 ×g and room temperature for 1 to 5 minutes to remove the solution in the tube. This operation is repeated once more, followed by further centrifugation at 10000 to 22000 ×g and room temperature for 1 to 5 minutes, and the tube is changed to a fresh one, followed by addition of 20 to 60 µL of sterile water and centrifugation at 10000 to 22000 ×g and room temperature for 1 to 3 minutes to recover a flow-through solution.

The above obtained solution or flow-through solution treated in the step 1 according to the present invention (treatment with a methylation-sensitive restriction enzyme) is subjected to the step 2 according to the present invention.

### (4) Step 2 according to amplification method according to embodiment of present invention

To 1 to 3 µL of the solution or flow-through solution treated in the step 1 according to the present invention, 4 to 6 µL of sterile water, 5 to 12 µL of a reagent for digital PCR containing a nucleic acid synthesis substrate, a nucleic acid synthesis enzyme, and the like [for example, ddPCR™Supermix for Probes (manufactured by Bio-Rad Laboratories Ltd.)], 0.5 to 1.5 µL of 1 to 20 µM forward primer (for example, hcf-F-fox), 0.5 to 1.5 µL of 1 to 20 µM reverse primer (for example, hcf-R-fox), 0.5 to 1 µL of 1 to 10 µM probe (for example, hcf-Probe fox), and 0.5 to 1.5 µL of 1 to 50 units/µL methylation-sensitive restriction enzyme (for example, HapII or HpaII) are added to prepare a reaction solution for digital PCR. Next, the reaction solution for digital PCR is set in a nucleic acid amplification device [for example, a thermal cycler (manufactured by Bio-Rad Laboratories Ltd.)] and incubated at 30°C to 40°C for 30 to 60 minutes. Thereafter, droplets are produced by a droplet producing device [for example, QX200 Droplet Generator (manufactured by Bio-Rad Laboratories Ltd.) or Automated Droplet Generator (manufactured by Bio-Rad Laboratories Ltd.)]. Subsequently, the droplets are dispensed into a well plate (for example, a 96-well plate). Then, using a nucleic acid amplification device [for example, a thermal cycler (manufactured by Bio-Rad Laboratories Ltd.)], the droplets are heated at a temperature of 90°C to 97°C for 5 to 15 minutes, followed by heating for 30 to 50 cycles with (1) 90°C to 95°C for 25 to 30 seconds and (2) 60°C to 65°C for 30 to 90 seconds as one cycle, whereby it is possible to amplify a methylated analysis target region (for example, a region consisting of the base sequence represented by SEQ ID NO: 11).

### (B) Case where specific region of DNA present in tissue, cell, whole blood, urine, or the like (for example, region consisting of the base sequence represented by SEQ ID NO: 1) is set as analysis target region.

### (1) Extraction of DNA

DNA (for example, genomic DNA) is extracted from a sample (for example, a cell), using, for example, a nucleic acid extraction kit [for example, QuickGene SP kit DNA tissue (manufactured by Kurabo Industries Co., Ltd.)].

### (2) Fragmentation step according to present invention

To the DNA obtained in the section (1) are added 3 to 7 µL of a buffer solution (for example, a Tris buffer solution) in the fragmentation step according to the present invention and 0.5 to 1.5 µL of a restriction enzyme (for example, BamHI or/and HindIII) in the fragmentation step of 0.5 to 25 units/µL, which is then adjusted to a volume of 30 to 60 µL with sterile water. Thereafter, the reaction mixture is reacted at 20°C to 50°C for 40 to 80 minutes to obtain a solution treated in the fragmentation step according to the present invention.

Further, if necessary, the solution treated in the fragmentation step according to the present invention may be subjected to the purification step according to the present invention as follows.

That is, 200 to 300 µL of a protein denaturing agent (for example, guanidine hydrochloride) and 200 to 300 µL of a buffer solution (for example, a Tris buffer solution) with a pH of 5 to 8 are added to and mixed with the solution treated in the fragmentation step according to the present invention. Then, the mixture is transferred to, for example, a column filled with a filler [for example, ECONOSPIN (manufactured by Gene Design Inc.)] and centrifuged at 10000 to 22000 ×g and room temperature for 1 to 3 minutes to remove the solution in the tube. Thereafter, 500 to 700 µL of a buffer solution (for example, a Tris buffer solution) with a pH of 5 to 8 and 500 to 700 µL of an alcohol (for example, ethanol) are added thereto, followed by centrifugation at 10000 to 22000 ×g and room temperature for 1 to 5 minutes, and the tube is changed to a fresh one, followed by addition of 20 to 40 µL of a buffer solution (for example, a Tris buffer solution) and centrifugation at 10000 to 22000 ×g and room temperature for 1 to 3 minutes to recover a flow-through solution.

The above obtained solution or flow-through solution treated in the fragmentation step according to the present invention is subjected to the step 1 according to the present invention.

### (3) Step 1 according to present invention

### (3)-1 Treatment with S1 nuclease

To 20 to 40 µL of the solution or flow-through solution treated in the fragmentation step according to the present invention are added 15 to 25 µL of sterile water, 2 to 6 µL of a buffer solution (for example, a sodium acetate buffer solution) in the step 1 according to the present invention, and 0.5 to 1.5 µL of 1 to 200 units/µL S1 nuclease, followed by reaction at 10°C to 37°C for 10 to 20 minutes. Thereafter, 0.5 to 1.5 µL of a reaction stop solution (for example, 0.5 M EDTA) is added thereto, followed by elution with 25 to 35 µL of a buffer solution (for example, a Tris buffer solution) to obtain a solution treated in the step 1 according to the present invention (treatment with S1 nuclease).

Further, if necessary, the solution treated in the step 1 according to the present invention may be subjected to the purification step according to the present invention as follows.

That is, 200 to 300 µL of a protein denaturing agent (for example, guanidine hydrochloride) and 200 to 300 µL of a buffer solution (for example, a Tris buffer solution) with a pH of 5 to 8 are added to and mixed with 10 to 20 µL of the solution treated in the step 1 according to the present invention (treatment with S1 nuclease). Then, the mixture is transferred to, for example, a column filled with a filler [for example, ECONOSPIN (manufactured by Gene Design Inc.)] and centrifuged at 10000 to 22000 ×g and room temperature for 1 to 3 minutes to remove the solution in the tube. Thereafter, 500 to 700 µL of a buffer solution (for example, a Tris buffer solution) with a pH of 5 to 8 and 500 to 700 µL of an alcohol (for example, ethanol) are added thereto, followed by centrifugation at 10000 to 22000 ×g and room temperature for 1 to 5 minutes, and the tube is changed to a fresh one, followed by addition of 20 to 40 µL of a buffer solution (for example, a Tris buffer solution) and centrifugation at 10000 to 22000 ×g and room temperature for 1 to 3 minutes to recover a flow-through solution.

The above obtained solution or flow-through solution treated in the step 1 according to the present invention (treatment with S1 nuclease) is subjected to the step 1 according to the present invention (treatment with a methylation-sensitive restriction enzyme).

### (3)-2 Treatment with methylation-sensitive restriction enzyme

To 20 to 30 µL of the solution or flow-through solution treated in the step 1 according to the present invention (treatment with S1 nuclease) are added 5 to 15 µL of sterile water, 2 to 6 µL of a buffer solution (for example, a Tris buffer solution) in the step 1 according to the present invention, and 0.5 to 1.5 µL of 1 to 50 units/µL methylation-sensitive restriction enzyme (for example, HapII, HpaII, or SacII), followed by reaction at 20°C to 50°C for 60 to 150 minutes to obtain a solution treated in the step 1 according to the present invention (treatment with a methylation-sensitive restriction enzyme).

Further, if necessary, the solution treated in the step 1 according to the present invention (treatment with a methylation-sensitive restriction enzyme) may be subjected to the purification step according to the present invention as follows.

That is, 200 to 300 µL of a protein denaturing agent (for example, guanidine hydrochloride) and 200 to 300 µL of a buffer solution (for example, a Tris buffer solution) with a pH of 5 to 8 are added to and mixed with the solution treated in the step 1 according to the present invention (treatment with a methylation-sensitive restriction enzyme). Then, the mixture is transferred to, for example, a column filled with a filler [for example, ECONOSPIN (manufactured by Gene Design Inc.)] and centrifuged at 10000 to 22000 ×g and room temperature for 1 to 3 minutes to remove the solution in the tube. Thereafter, 500 to 700 µL of a buffer solution (for example, a Tris buffer solution) with a pH of 5 to 8 and 500 to 700 µL of an alcohol (for example, ethanol) are added thereto, followed by centrifugation at 10000 to 22000 ×g and room temperature for 1 to 5 minutes, and the tube is changed to a fresh one, followed by addition of 20 to 40 µL of a buffer solution (for example, a Tris buffer solution) and centrifugation at 10000 to 22000 ×g and room temperature for 1 to 3 minutes to recover a flow-through solution.

The above obtained solution or flow-through solution treated in the step 1 according to the present invention (treatment with a methylation-sensitive restriction enzyme) is subjected to the step 2 according to the present invention.

### (4) Step 2 according to present invention

To 0.5 to 1.5 µL of the solution or flow-through solution treated in the step 1 according to the present invention, 15 to 20 µL of sterile water, 2 to 3 µL of a buffer solution for PCR reaction (for example, a Tris buffer solution), 1.5 to 2.5 µL of a nucleic acid synthesis substrate, 0.1 to 0.3 µL of 1 to 10 units/µL nucleic acid synthesis enzyme, 0.5 to 1.5 µL of 5 to 15 µM forward primer (for example, h-F-fox), and 0.5 to 1.5 µL of 5 to 15 µM reverse primer (for example, h-R-fox) are added to prepare a reaction solution for PCR. Then, using a nucleic acid amplification device [for example, a thermal cycler (manufactured by Bio-Rad Laboratories Ltd.)], the reaction solution for PCR is heated at a temperature of 92°C to 96°C for 1 to 3 minutes, followed by heating for 30 to 60 cycles with (1) 92°C to 98°C for 2 to 10 seconds and (2) 65°C to 72°C for 10 to 20 seconds as one cycle, and further heating at 65°C to 72°C for 50 to 70 seconds, whereby it is possible to amplify a methylated analysis target region (for example, a region consisting of the base sequence represented by SEQ ID NO: 1).

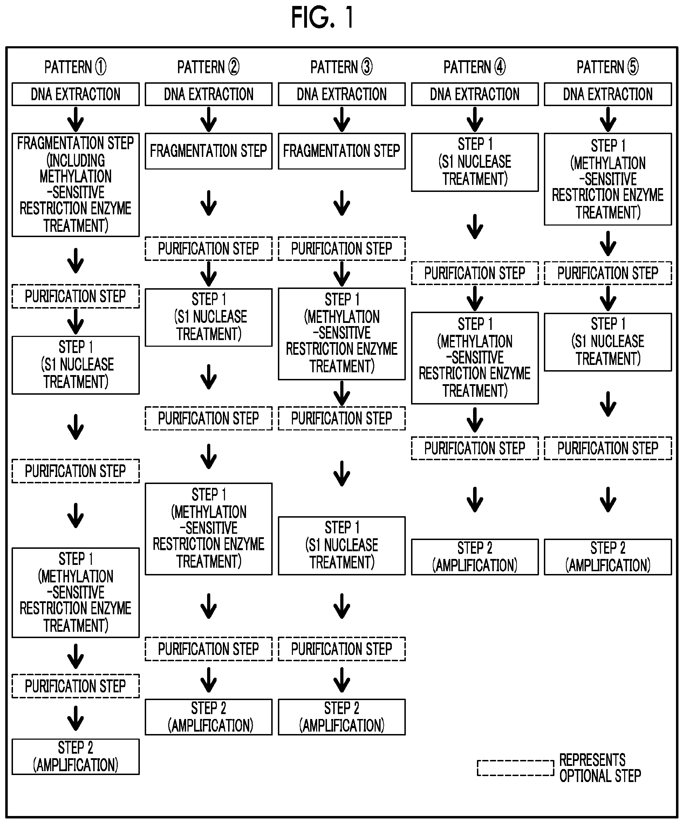

Fig. 1 shows a flow chart of the amplification method according to the embodiment of the present invention divided into various patterns. Examples of the amplification method according to the embodiment of the present invention include the methods of Patterns 1 to 5, among which Patterns 1 to 3 are more preferable, Pattern 1 or 2 is still more preferable, and Pattern 1 is particularly preferable.

### Methylation determination method according to embodiment of present invention

The methylation determination method according to the embodiment of the present invention is to determine whether or not the analysis target region is methylated based on the results obtained by the amplification method according to the embodiment of the present invention.

That is, the methylation determination method according to the embodiment of the present invention is for confirmation of the amplification product and determination as to whether or not the analysis target region is methylated, following the amplification method of the present invention, and involves (1) a step 1 of treating with S1 nuclease and a methylation-sensitive restriction enzyme, (2) a step 2 of amplifying an analysis target region of the double-stranded DNA treated in the step 1, (3) a step 3 of confirming the presence or absence of the amplification product obtained in the step 2, and (4) a step 4 of determining whether or not the analysis target region is methylated based on the results of the step 3.

The steps (steps 1 and 2) before the step 3 until the amplification product is obtained are the same as the amplification method according to the embodiment of the present invention, and specific examples, preferred examples, and the like thereof are also the same. The step 3 and the following will be described in detail below.

### Step 3

The step 3 according to the present invention is a step of confirming the presence or absence of the amplification product obtained in the step 2 according to the present invention. That is, the step 3 according to the present invention is a step of confirming the presence or absence of the methylated analysis target region since the methylated analysis target region is amplified in the step 2 according to the present invention.

The method of confirming the presence or absence of the amplification product in the step 3 according to the present invention is not particularly limited as long as it is a method commonly carried out in the art, and may be carried out based on a method known per se.

Specific examples thereof include (a) a method of confirming by electrophoresis, and (b) a method of confirming with a fluorescence reader.

### (a) Method of confirming by electrophoresis

The method of confirming by electrophoresis is a method of separating and confirming the amplification product obtained in the step 2 according to the present invention by electrophoresis. Specific methods thereof include (a-1) a labeled primer method, (a-2) an intercalator method, and (a-3) a labeled probe method.

### (a-1) Labeled primer method

The labeled primer method is a method in which "the step 2 according to the present invention is carried out using a primer pair containing a labeled primer in which at least one of the primers according to the present invention is labeled with a labeling substance, the resulting amplification product is then separated by electrophoresis, and the label in the amplification product is detected to confirm the presence or absence of the amplification product obtained in the step 2 according to the present invention".

In the present specification, "detecting the label" means directly or indirectly measuring the labeling substance based on the properties of the labeling substance.

The electrophoresis in the labeled primer method may be any electrophoresis as long as it is based on a method of migrating at different speeds or migrating at different distances depending on the charge intensity of the substance. Specific examples thereof include agarose gel electrophoresis, acrylamide gel electrophoresis, and capillary electrophoresis, among which agarose gel electrophoresis or capillary electrophoresis is more preferable.

The agarose gel electrophoresis method may be carried out based on the method described in, for example, "Bio-Experiment Illustrated II, Fundamentals of Gene Analysis, 2006, pp. 53 to 56". In addition, the capillary electrophoresis may be carried out based on the method described in, for example, WO2007/027495A, WO2011/118496A, or WO2008/075520A.

### (a-2) Intercalator method

The intercalator method is a method in which "the step 2 according to the present invention is carried out using the primer pair according to the present invention, the resulting amplification product is separated by electrophoresis, the amplification product is then stained with an intercalator, and fluorescence derived from the intercalator is detected to confirm the presence or absence of the amplification product obtained in the step 2 according to the present invention".

Examples of the electrophoresis in the intercalator method are the same ones as in (a-1) Labeled primer method, and preferred ones thereof are also the same.

In addition, the intercalator in the intercalator method may be any intercalator commonly used in the art. Specific examples thereof include intercalators of (1) to (5) and intercalator analogues of (6) and (7) below:
(1) ethidium compounds [for example, ethidium bromide, ethidium homodimer 1 (EthD-1), ethidium homodimer 2 (EthD-2), ethidium bromide monoazide (EMA), and dihydroethidium];
(2) acridine dyes (for example, acridine orange);
(3) iodine compounds (propidium iodide, hexidium iodide, and the like), and cyanine dimer dyes [for example, POPO-1, BOBO-1, YOYO-1, TOTO-1, JOJO-1, POPO-3, LOLO-1, BOBO-3, YOYO-3, and TOTO-3 (all manufactured by Molecular Probes, Inc.)];
(4) cyanine monomer dyes [for example, PO-PRO-1, BO-PRO-1, YO-PRO-1, TO-PRO-1, JO-PRO-1, PO-PRO-3, LO-PRO-1, BO-PRO-3, YO-PRO-3, TO-PRO-3, and TO-PRO-5 (all manufactured by Molecular Probes, Inc.)];
(5) SYTOX dyes [for example, SYBR Gold, SYBR Green I, SYBR Green II, SYTOX Green, SYTOX Blue, and SYTOX Orange (all manufactured by Molecular Probes, Inc.)], and GelRed dyes [for example, GelRed Nucleic Acid Gel Stain (manufactured by Wako Pure Chemical Industries, Ltd.)];
(6) compounds which bind to minor groove of DNA double helix [for example, 4',6-diamidino-2-phenylindole (DAPI: manufactured by Molecular Probes, Inc.)]; and
(7) compounds which specifically bind to an adenine-thymine (A-T) sequence [for example, pentahydrate (bis-benzimide) (Hoechst 33258: manufactured by Molecular Probes, Inc.), trihydrochloride (Hoechst 33342: manufactured by Molecular Probes, Inc.), and bisbenzimide dyes (Hoechst 34580: manufactured by Molecular Probes, Inc.), and the like]. Among them, SYTOX dyes [for example, SYBR Gold, SYBR Green I, SYBR Green II, SYTOX Green, SYTOX Blue, and SYTOX Orange (all manufactured by Molecular Probes, Inc.)], GelRed dyes [for example, GelRed Nucleic Acid Gel Stain (manufactured by Wako Pure Chemical Industries, Ltd.)] are more preferable, and GelRed dyes [for example, GelRed Nucleic Acid Gel Stain (manufactured by Wako Pure Chemical Industries, Ltd.)] are particularly preferable.

### (a-3) Labeled probe method

The labeled probe method is a method in which "the step 2 according to the present invention is carried out using the primer pair according to the present invention, the resulting amplification product is separated by electrophoresis, the amplification product is then subjected to a heat treatment to make it single-stranded, the resulting single-stranded amplification product is hybridized to a probe labeled with a labeling substance and having a base sequence complementary to the base sequence of the amplification product to obtain a hybrid product, and the label in the hybrid product is detected to confirm the presence or absence of the amplification product obtained in the step 2 according to the present invention".

Examples of the electrophoresis in the labeled probe are the same ones as in (a-1) Labeled primer method, and preferred ones thereof are also the same.

### (b) Method of detecting with fluorescence reader

The method of detecting with a fluorescence reader is a method in which "the step 2 according to the present invention using the primer pair and the probe according to the present invention is carried out and then the fluorescence derived from each droplet is detected with a fluorescence reader to confirm the presence or absence of the amplification product obtained in the step 2 according to the present invention".

As the fluorescence reader in the method of detecting with a fluorescence reader, one attached to a digital PCR device [for example, QX200 Droplet Reader (manufactured by Bio-Rad Laboratories Ltd.)] may be used.

As described above, it can be confirmed whether or not the methylated analysis target region has been amplified by carrying out the step 3 according to the present invention.

### Step 4

The step 4 according to the methylation determination method according to the embodiment of the present invention is a step of determining whether or not the analysis target region is methylated based on the results of the step 3 according to the present invention.

That is, the determination of whether or not the analysis target region according to the present invention is methylated is made based on the results of confirming the presence or absence of the amplification product amplified in the step 2 according to the present invention. In the case where the analysis target region is methylated, an amplification product is obtained since the analysis target region is amplified in the step 2 according to the present invention. On the other hand, in the case where the analysis target region is not methylated, an amplification product cannot be obtained since the analysis target region is not amplified in the step 2 according to the present invention. This is due to the properties of the methylation-sensitive restriction enzyme in the step 1 according to the present invention.

That is, in the case where the analysis target region is methylated, the analysis target region is not cleaved even in the case where it is treated with a methylation-sensitive restriction enzyme, so that the analysis target region is amplified in the amplification reaction of the step 2 according to the present invention, and therefore the amplification product is confirmed in the step 3 according to the present invention.

On the other hand, in the case where the analysis target region is not methylated, the analysis target region is cleaved by a methylation-sensitive restriction enzyme, so that the analysis target region is not amplified in the step 2 according to the present invention, and therefore no amplification product is confirmed in the step 3 according to the present invention.

Accordingly, (a) in the case of carrying out the method of confirming by electrophoresis in the step 3 according to the present invention, (i) it can be determined that the analysis target region is methylated in the case where an amplification product is confirmed, and (ii) it can be determined that the analysis target region is not methylated in the case where no amplification product is confirmed.

On the other hand, (b) in the case of carrying out the method of confirming by a fluorescence reader in the step 3 according to the present invention, (i) it can be determined that the analysis target region is methylated in the case where at least one droplet emitting fluorescence exceeding a preset threshold value is confirmed, and (ii) it can be determined that the analysis target region is not methylated in the case where no droplet emitting fluorescence exceeding a preset threshold value is confirmed.

Incidentally, the threshold value may be determined according to the threshold setting function of the fluorescence reader [for example, QX200 Droplet Reader (manufactured by Bio-Rad Laboratories Ltd.)] or may be determined from the results of amplifying the analysis target region.

As described above, it can be determined whether or not the analysis target region is methylated by carrying out the step 4 according to the methylation determination method according to the embodiment of the present invention.

In addition, the methylation determination method according to the embodiment of the present invention is a very convenient method since it is capable of determining whether or not the analysis target region is methylated by confirming the presence or absence of the amplification product as described above.

### Specific examples of methylation determination method according to embodiment of present invention

Hereinafter, specific examples of the methylation determination method according to the embodiment of the present invention will be described separately in the case (A) where a specific region of DNA present in a body fluid such as serum, plasma, or urine is set as an analysis target region, and in the case (B) where a specific region of DNA present in tissue, cell, whole blood, urine, or the like is set as an analysis target region.

### (A) In case where specific region of DNA present in a body fluid such as serum, plasma, or urine (for example, region consisting of base sequence represented by SEQ ID NO: 11) is set as analysis target region

### (1) Extraction of DNA, (2) Fragmentation step according to present invention, (3) Step 1 according to present invention, and (4) Step 2 according to present invention are as described in the section of "Specific examples of amplification method according to embodiment of present invention".

### (5) Step 3 according to present invention

The presence or absence of the amplification product amplified in the step 2 according to the present invention can be confirmed by detecting the fluorescence in the amplification product amplified in the step 2 according to the present invention in each droplet by a fluorescence reader [for example, QX200 Droplet Reader (manufactured by Bio-Rad Laboratories Ltd.)].

### (6) Step 4 according to methylation determination method according to embodiment of present invention

It can be determined that "the analysis target region is methylated" in the case where at least one droplet emitting fluorescence exceeding a preset threshold value is confirmed as a result of confirming the presence or absence of the amplification product of the step 3 according to the present invention. On the other hand, it can be determined that "the analysis target region is not methylated" in the case where a droplet emitting fluorescence exceeding a preset threshold value is not confirmed.

### (B) In case where specific region of DNA present in tissue, cell, whole blood, urine, or the like (for example, region consisting of base sequence represented by SEQ ID NO: 1) is set as analysis target region

### (1) Extraction of DNA, (2) Fragmentation step according to present invention, (3) Step 1 according to present invention, and (4) Step 2 according to present invention are as described in the section of "Specific examples of amplification method according to embodiment of present invention".

### (5) Step 3 according to present invention

The amplification product amplified in the step 2 according to the present invention is subjected to 0.5% to 5% agarose gel electrophoresis together with a molecular weight marker. This is followed by staining with an intercalator [for example, GelRed Nucleic Acid Gel Stain (manufactured by Wako Pure Chemical Industries, Ltd.)] and then irradiation with UV to detect the amplification product, so that the presence or absence of the amplification product amplified in the step 2 according to the present invention can be confirmed.

### (6) Step 4 according to methylation determination method according to embodiment of present invention

It can be determined that "the analysis target region is methylated" in the case where the amplification product is confirmed as a result of confirming the presence or absence of the amplification product of the step 3 according to the present invention. On the other hand, it can be determined that "the analysis target region is not methylated" in the case where the amplification product is not confirmed.

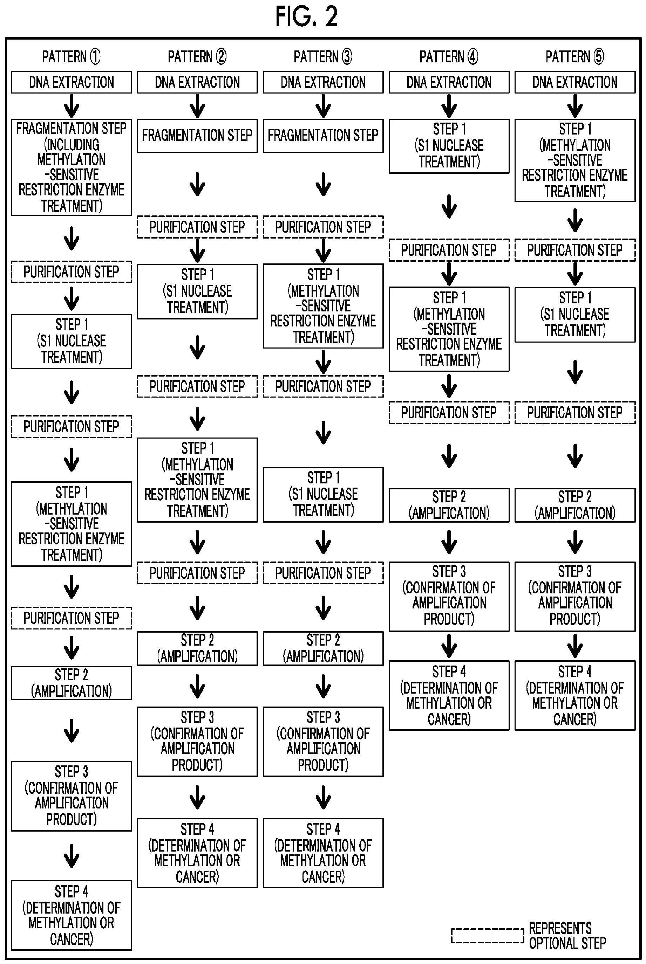

Fig. 2 shows a flow chart of the methylation determination method according to the embodiment of the present invention divided into various patterns. Examples of the methylation determination method according to the embodiment of the present invention include the methods of Patterns 1 to 5, among which Patterns 1 to 3 are more preferable, Pattern 1 or 2 is still more preferable, and Pattern 1 is particularly preferable.

After carrying out the methylation determination method according to the embodiment of the present invention, bisulfite sequencing analysis, microarray analysis, or the like may be carried out as necessary. Thus, not only whether or not the analysis target region according to the present invention is methylated but also which CpG sequence is methylated can be determined.

### Reagent for determining methylation of present invention

The reagent for determining methylation of the present invention contains S1 nuclease and a methylation-sensitive restriction enzyme.

Specific examples and preferred examples of the methylation-sensitive restriction enzyme used in the reagent for use in the determination of methylation of the present invention are as described above.

Further, one or more of the following reagents commonly used in the art may be added to the reagent for determination of the present invention:
a) a column for DNA purification [for example, ECONOSPIN (manufactured by Gene Design Inc.)];
b) a reagent for amplification reaction such as PCR (for example, one or a plurality of primers, probes, nucleic acid synthesis substrates, or nucleic acid synthesis enzymes);
c) a restriction enzyme for fragmentation;
d) a reagent for confirming a nucleic acid amplification product [for example, agarose gel, loading buffer solution, or reagent for staining (GelRed stain, ethidium bromide, or the like)]; and
e) a reagent for DNA extraction [for example, QuickGene SP kit DNA tissue (manufactured by Kurabo Industries Ltd.), NucleoSpin™ Plasma XS (manufactured by Macherey-Nagel Inc.), or MagMAXTM Cell-Free DNA Isolation Kit (manufactured by Thermo Fisher Scientific Inc.)].

In addition, the reagent for determining methylation of the present invention may contain instructions for carrying out the methylation determination method according to the embodiment of the present invention and the like. The term "instructions" refers to an instruction manual, an attached sentence, a pamphlet (leaflet), or the like relating to the reagent for use in the determination of methylation of the present invention, in which the characteristics, principle, operation procedure, and the like of the methylation determination method of the present invention are substantially described by sentences or views.

As described above, the methylation determination method according to the embodiment of the present invention can be carried out with convenience and high accuracy in a short period of time with the reagent for use in the determination of methylation of the present invention.

### Method for determining cancer according to embodiment of present invention

The method for determining cancer according to the embodiment of the present invention is a method for determining cancer on a subject for which it is determined whether or not there is a cancer, based on the results obtained by the amplification method according to the embodiment of the present invention. That is, the method for determining cancer according to the embodiment of the present invention carries out a step of confirming an amplification product and a step of determining cancer subsequent to the amplification method of the present invention, and includes (1) a step 1 of treating DNA containing an analysis target region with S1 nuclease and a methylation-sensitive restriction enzyme, (2) a step 2 of amplifying the analysis target region of the double-stranded DNA treated in the step 1, (3) a step 3 of confirming the presence or absence of the amplification product obtained in the step 2, and (4) a step 4 of determining cancer based on the results of the step 3.

The steps (steps 1 and 2) before the step 3 until the amplification product is obtained are the same as the amplification method according to the embodiment of the present invention, and specific examples, preferred examples, and the like thereof are also the same. In addition, the step 3 of confirming the amplification product is the same as in the methylation determination method according to the embodiment of the present invention, and specific examples, preferred examples, and the like thereof are also the same.

### Cancer

The cancer according to the method for determining cancer according to the embodiment of the present invention is a disorder or disease characterized by uncontrolled cell division, direct proliferation or metastasis to adjacent tissues through invasion, and the like. Specific examples of the cancer to be determined by the determination method according to the embodiment of the present invention include cell carcinoma, adenocarcinoma, lymphoma, leukemia, sarcoma, mesothelioma, neuroma, osteosarcoma, germinoma, prostate cancer, lung cancer, breast cancer, colorectal cancer, gastrointestinal cancer, bladder cancer, pancreatic cancer, endometrial cancer, cervical cancer, ovarian cancer, melanoma, brain cancer, testicular cancer, kidney cancer, skin cancer, thyroid cancer, head and neck cancer, liver cancer, esophageal cancer, gastric cancer, colon cancer, bone marrow carcinoma, neuroblastoma, and retinoblastoma, among which lung cancer, breast cancer, pancreatic cancer, liver cancer, gastric cancer, or colon cancer is more preferable.

### Step 4

The step 4 according to the method for determining cancer according to the embodiment of the present invention is a step of determining cancer based on the results of the step 3 according to the present invention. That is, the step of determining cancer is carried out based on the results of confirming the presence or absence of the amplification product amplified in the step 2 according to the present invention.

The determination of cancer in the step 4 according to the method for determining cancer according to the embodiment of the present invention is made using data obtained from the results of the steps 1 to 3 according to the present invention. That is, in the case where a target amplification product amplified in the step 2 according to the present invention is detected and the result that the amplification product is detected is obtained, a determination of cancer is made on a subject for which it is determined whether or not there is a cancer.

The subject for which it is determined whether or not there is a cancer is specified according to the sample derived from DNA containing an analysis target region.

For example, in the case where the sample derived from DNA containing an analysis target region is a tissue, a cell, or the like, the subject for which it is determined whether or not there is a cancer is the tissue, cell, or the like itself, furthermore, an animal from which the tissue, cell, or the like is derived. That is, in the case where the sample derived from DNA containing an analysis target region is a tissue, a cell, or the like, it is determined whether or not the tissue, cell, or the like itself is cancerized, and further it is determined whether or not an animal from which the tissue, cell, or the like is derived is affected by cancer or whether or not cancer cells are present in the animal.

On the other hand, in the case where the sample derived from DNA containing an analysis target region is a body fluid such as whole blood, serum, plasma, or urine, the subjects for which it is determined whether or not there is a cancer are as follows.

In the case where the DNA containing the analysis target region is derived from a cell (a blood cell such as white blood cell, a cell derived from each tissue, CTC, or the like) in body fluid such as whole blood, serum, plasma, or urine, the subject is the cell itself, and furthermore an animal from which the cell is derived. That is, in the case where the DNA containing the analysis target region is derived from a cell (a blood cell such as white blood cell, a cell derived from each tissue, CTC, or the like) in a body fluid such as whole blood, serum, plasma, or urine, it is determined whether or not the cancer itself has been cancerized and furthermore it is determined whether or not an animal from which the cell is derived is affected by cancer or whether or not cancer cells are present in the animal.

In addition, in the case where the DNA containing an analysis target region is derived from cfDNA, exosome, or the like in body fluid such as serum, plasma, or urine, the subject for which it is determined whether or not there is a cancer is an animal from which the body fluid such as serum, plasma, or urine is derived. That is, in the case where the DNA containing an analysis target region is derived from cfDNA, exosome, or the like in the body fluid such as serum, plasma, or urine, it is determined whether the animal from which the body fluid such as serum, plasma, or urine is derived is affected by cancer or whether or not cancer cells are present in the animal.

In the case where the subject for which it is determined whether or not there is a cancer is affected by cancer or cancer cells are present in the subject, an amplification product is obtained since the analysis target region is amplified in the step 2 according to the present invention. On the other hand, in the case where the subject for which it is determined whether or not there is a cancer is not affected by cancer or cancer cells are not present in the subject, no amplification product is detected since the analysis target region is not amplified in the step 2 according to the present invention. This is due to the properties of the methylation-sensitive restriction enzyme in the step 1 according to the present invention.

That is, in the case where the analysis target region is methylated, the analysis target region is not cleaved even in the case where it is treated with a methylation-sensitive restriction enzyme, so that the analysis target region is amplified in the amplification reaction of the step 2 according to the present invention, and therefore the amplification product is confirmed in the step 3 according to the present invention.

On the other hand, in the case where the analysis target region is not methylated, the analysis target region is cleaved by a methylation-sensitive restriction enzyme, so that the analysis target region is not amplified in the step 2 according to the present invention, and therefore no amplification product is confirmed in the step 3 according to the present invention.

Accordingly, (a) in the case of carrying out the method of confirming by electrophoresis in the step 3 according to the present invention, (i) it can be determined that the subject from which the analysis target region is derived and for which it is determined whether or not there is a cancer is affected by cancer or cancer cells are present in the subject in the case where an amplification product is confirmed, and (ii) it can be determined that the subject from which the analysis target region is derived and for which it is determined whether or not there is acancer is not affected by cancer or cancer cells are not present in the subject in the case where no amplification product is confirmed.

On the other hand, (b) in the case of carrying out the method of confirming by a fluorescence reader in the step 3 according to the present invention, (i) it can be determined that the subject from which the analysis target region is derived and for which it is determined whether or not there is a cancer is affected by cancer or cancer cells are present in the subject in the case where at least one droplet emitting fluorescence exceeding a preset threshold value is confirmed, and (ii) it can be determined that the subject from which the analysis target region is derived and for which it is determined whether or not there is a cancer is not affected by cancer or cancer cells are not present in the subject in the case where no droplet emitting fluorescence exceeding a preset threshold value is confirmed.

Furthermore, the threshold value may be determined according to the threshold setting function of the fluorescence reader [for example, QX200 Droplet Reader (manufactured by Bio-Rad Laboratories Ltd.)] or may be determined from the results of amplifying the analysis target region.

As described above, cancer can be determined by carrying out the step 4 according to the method for determining cancer according to the embodiment of the present invention.

As described above, the method for determining cancer according to the embodiment of the present invention is a very convenient method because it is only necessary to confirm the presence or absence of the amplification product.

### Specific examples of method for determining cancer according to embodiment of present invention

Hereinafter, specific examples of the method for determining cancer according to the embodiment of the present invention will be described separately in the case (A) where a specific region of DNA present in a body fluid such as serum, plasma, or urine is set as an analysis target region, and in the case (B) where a specific region of DNA present in tissue, cell, whole blood, urine, or the like is set as an analysis target region.

### (A) In case where specific region of DNA present in a body fluid such as serum, plasma, or urine (for example, region consisting of the base sequence represented by SEQ ID NO: 11) is set as analysis target region

**(1) Extraction of DNA, (2) Fragmentation step according to present invention, (3) Step 1 according to present invention, and (4) Step 2 according to present invention** are as described in the section of **"Specific examples of amplification method according to embodiment of present invention".** In addition, **(5) Step 3 according to present invention** is as described in the section of **"Specific examples of methylation determination method according to embodiment of present invention".**

### (6) Step 4 according to method for determining cancer according to embodiment of present invention

It can be determined that "the subject from which the analysis target region is derived and for which it is determined whether or not there is a cancer is affected by cancer or cancer cells are present in the subject" in the case where at least one droplet emitting fluorescence exceeding a preset threshold value is confirmed as a result of confirming the presence or absence of the amplification product of the step 3 according to the present invention. On the other hand, it can be determined that "the subject from which the analysis target region is derived and for which it is determined whether or not there is an occurrence of cancer is not affected by cancer or cancer cells are not present in the subject" in the case where at least one droplet emitting fluorescence exceeding a preset threshold value is not confirmed.

### (B) Case where specific region of DNA present in tissue, cell, whole blood, urine, or the like (for example, region consisting of base sequence represented by SEQ ID NO: 1) is set as analysis target region

**(1) Extraction of DNA, (2) Fragmentation step according to present invention, (3) Step 1 according to present invention, and (4) Step 2 according to present invention** are as described in the section of **"Specific examples of amplification method according to embodiment of present invention".** In addition, **(5) Step 3 according to present invention** is as described in the section of **"Specific examples of methylation determination method according to embodiment of present invention".**

### (6) Step 4 according to method for determining cancer according to embodiment of present invention

It can be determined that "the subject from which the analysis target region is derived and for which it is determined whether or not there is a cancer is affected by cancer or cancer cells are present in the subject" in the case where an amplification product is confirmed as a result of confirming the presence or absence of the amplification product of the step 3 according to the present invention. On the other hand, it can be determined that "the subject from which the analysis target region is derived and for which it is determined whether or not there is an occurrence of cancer is not affected by cancer or cancer cells are not present in the subject" in the case where no amplification product is confirmed.

Fig. 2 shows a flow chart of the method for determining cancer according to the embodiment of the present invention divided into various patterns. Examples of the method for determining cancer according to the embodiment of the present invention include the methods of Patterns 1 to 5, among which Patterns 1 to 3 are more preferable, Pattern 1 or 2 is still more preferable, and Pattern 1 is particularly preferable.

### Method for obtaining data for determining cancer of present invention

The method for obtaining data for determining cancer of the present invention (hereinafter, sometimes referred to simply as a method for obtaining data of the present invention) acquires data for determining cancer with respect to a subject for which it is determined whether or not there is a cancer based on the results obtained by the amplification method according to the embodiment of the present invention. That is, the method for obtaining data for determining cancer of the present invention carries out a step of confirming the presence or absence of an amplification product subsequent to the amplification method according to the embodiment of the present invention, and includes (1) a step 1 of treating DNA containing an analysis target region with S1 nuclease and a methylation-sensitive restriction enzyme, (2) a step 2 of amplifying the analysis target region of the double-stranded DNA treated in the step 1, and (3) a step 3 of confirming the presence or absence of the amplification product obtained in the step 2.

The steps (steps 1 and 2) before the step 3 until the amplification product is obtained are the same as the amplification method according to the embodiment of the present invention, and specific examples, preferred examples, and the like thereof are also the same. In addition, the step 3 of confirming the amplification product is the same as in the methylation determination method according to the embodiment of the present invention, and specific examples, preferred examples, and the like thereof are also the same.

### Reagent for determining cancer of present invention

The reagent for determining cancer of the present invention contains S1 nuclease and a methylation-sensitive restriction enzyme.

Specific examples and preferred examples of the methylation-sensitive restriction enzyme used in the reagent for determining cancer of the present invention are as described above.

Further, one or more of the following reagents commonly used in the art may be added to the reagent for determination of the present invention:
a) a column for DNA purification [for example, ECONOSPIN (manufactured by Gene Design Inc.)];
b) a reagent for nucleic acid amplification reaction such as PCR (for example, one or a plurality of primers, probes, nucleic acid synthesis substrates, or nucleic acid synthesis enzymes);
c) a restriction enzyme for fragmentation;
d) a reagent for confirming a nucleic acid amplification product [for example, agarose gel, loading buffer solution, or reagent for staining (GelRed stain, ethidium bromide, or the like)]; and
e) a reagent for DNA extraction [for example, QuickGene SP kit DNA tissue (manufactured by Kurabo Industries Ltd.), NucleoSpin™ Plasma XS (manufactured by Macherey-Nagel Inc.), or MagMAX™ Cell-Free DNA Isolation Kit (manufactured by Thermo Fisher Scientific Inc.)].

In addition, the reagent for determining cancer of the present invention may contain instructions for carrying out the method for determining cancer according to the embodiment of the present invention and the like. The term "instructions" refers to an instruction manual, an attached sentence, a pamphlet (leaflet), or the like relating to the reagent for determining cancer of the present invention, in which the characteristics, principle, operation procedure, and the like of the method for determining cancer according to the embodiment of the present invention are substantially described by sentences or views.

As described above, the method for determining cancer according to the embodiment of the present invention can be carried out with convenience and high accuracy in a short period of time with the reagent for determining cancer of the present invention.

### Marker for determining cancer of present invention

The marker for determining cancer of the present invention (hereinafter, sometimes referred to simply as a marker of the present invention) is an amplification product obtained by carrying out the amplification method according to the embodiment of the present invention. The presence or absence of the marker (amplification product) makes it possible to determine cancer on a subject from which the analysis target region is derived and for which it is determined whether or not there is a cancer. That is, the marker is obtained by carrying out (1) a step 1 of treating DNA containing an analysis target region with S1 nuclease and a methylation-sensitive restriction enzyme, and (2) a step 2 of amplifying the analysis target region of the double-stranded DNA treated in the step 1.

The steps (steps 1 and 2) until the amplification product is obtained are the same as the amplification method according to the embodiment of the present invention, and specific examples, preferred examples, and the like thereof are also the same. In addition, the cancer and the subject for which it is determined whether or not there is a cancer are the same as in the method for determining cancer according to the embodiment of the present invention, and specific examples, preferred examples, and the like thereof are also the same.

### Method for detecting marker of present invention

The method for detecting the marker of the present invention is to detect the marker of the present invention by a method known per se. The marker is preferably detected by the method described in the step 3 of the present invention.

As described above, the method for determining cancer according to the embodiment of the present invention can be carried out with convenience and high accuracy in a short period of time by using the marker of the present invention.

Hereinafter, the present invention will be described in detail with reference to Examples and the like, but the present invention is not limited by these Examples and the like.

### Examples

### Experimental Example 1: Confirmation of methylation state of DNAs derived from various normal cells and various cancer cells

The methylation state on DNAs derived from various normal cells and various cancer cells described below was confirmed by bisulfite sequencing analysis.

### (1) Setting conditions

Samples and confirmation regions of methylation state were set as follows.

### • Samples

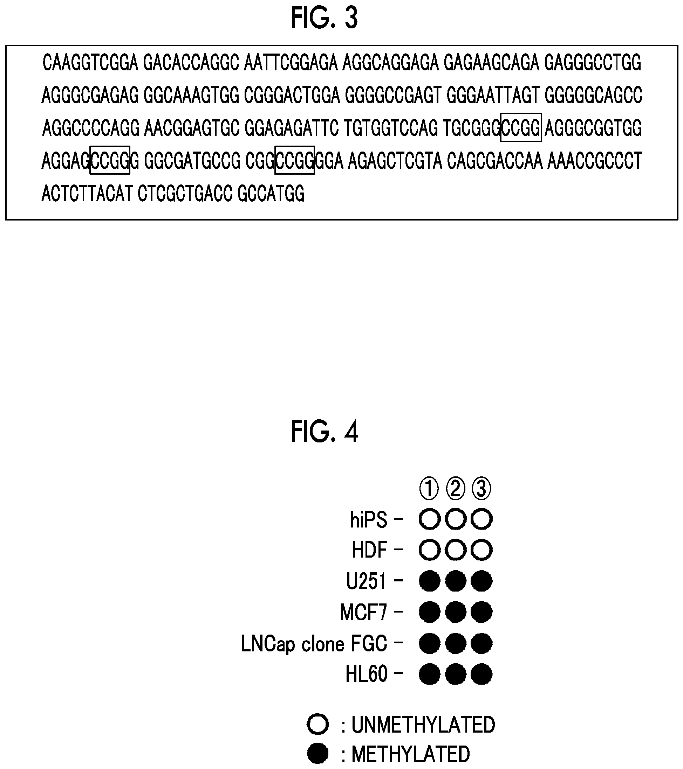

Human induced pluripotent stem cells (hiPS): normal cells
Normal fibroblasts (HDF): normal cells
Glioblastoma cells (U251-MG-P1): cancer cells
Mammary carcinoma cells (MCF-7): cancer cells
Prostate cancer cells (LNCap clone FGC): cancer cells
Myeloid leukemia cells (HL 60): cancer cells
• Confirmation region of methylation state
Boxed three sequences containing a CpG sequence (CCGG), shown in Fig. 3 and present in a region (267 base pairs) consisting of the base sequence represented by SEQ ID NO: 1 in the promoter region of the human-derived FOXB2 gene

### (2) Extraction of genomic DNA

Genomic DNA was extracted from each of the above cells using QuickGene SP kit DNA tissue (manufactured by Kurabo Industries Ltd.).

### (3) Bisulfite reaction

400 ng of each of the genomic DNAs obtained in the section (2) was dissolved in 10 µL of distilled water. Thereafter, using the Episight Bisulfite Conversion kit (manufactured by Wako Pure Chemical Industries, Ltd.), each of the solutions was subjected to a bisulfite reaction according to the method described in the kit.

### (4) PCR amplification reaction

To 1 µL of each of the bisulfite reaction product-containing solutions obtained in the section (3), 17.3 µL of distilled water, 2.5 µL of 10 × buffer for Blend Taq (manufactured by Toyobo Co., Ltd.), 2 µL of 2 mM dNTPs (manufactured by Toyobo Co., Ltd.), 0.2 µL of 2.5 units/µL Blend Taq-Plus (manufactured by Toyobo Co., Ltd.), 1 µL of 10 µM forward primer [GGAATTAGTGGGGGCAGCCAGGCCCCAGG (SEQ ID NO: 7)], and 1 µL of 10 µM reverse primer [CCCAAAAACTACCCTTACCAAACTAATC (SEQ ID NO: 8)] were added and mixed to prepare a reaction solution for PCR.

Next, each of the reaction solutions for PCR was set in a thermal cycler (manufactured by Bio-Rad Laboratories Ltd.), and a PCR amplification reaction was carried out under the following reaction conditions.
* Reaction conditions
94°C for 2 minutes
↓
36 cycles of 94°C for 10 seconds → 55°C for 10 seconds → 72°C for 20 seconds as one cycle
↓
72°C for 2 minutes

### (5) Cloning of PCR amplification product

Each of the PCR amplification products obtained in the section (4) was electrophoresed on a 1.5% agarose gel. After staining with GelRed Nucleic Acid Gel Stain (manufactured by Wako Pure Chemical Industries, Ltd.) subsequent to the electrophoresis, each of the PCR amplification products was recovered using a QIAquick Gel Extraction kit (manufactured by Qiagen AG).

### (6) PCR amplification reaction for adding restriction enzyme recognition site to PCR amplification product

To 1 µL of each of the PCR amplification product-containing solutions obtained in the section (5), 17.3 µL of distilled water, 2.5 µL of 10 × buffer for Blend Taq (manufactured by Toyobo Co., Ltd.), 2 µL of 2 mM dNTPs (manufactured by Toyobo Co., Ltd.), 0.2 µL of 2.5 units/µL Blend Taq-Plus (manufactured by Toyobo Co., Ltd.), 1 µL of 10 µM forward primer, and 1 µL of 10 µM reverse primer were added and mixed to prepare a reaction solution for PCR.

The forward primer is obtained by adding a restriction enzyme (HindIII) recognition site (underlined portion in the sequence) to the forward primer of the section (4), and consists of the following base sequence. The reverse primer is obtained by adding a restriction enzyme (BamHI) recognition site (underlined portion in the sequence) to the reverse primer of the section (4) and consists of the following base sequence.
Forward primer: TTACCATAAGCTTGGAATTAGTGGGGGCAGCCAGGCCCCAGG (SEQ ID NO: 9)
Reverse primer: TAATTAAGGATCCCCCAAAAACTACCCTTACCAAACTAATC (SEQ ID NO: 10)

Next, each of the reaction solutions for PCR was set in a thermal cycler (manufactured by Bio-Rad Laboratories Ltd.), and a PCR amplification reaction was carried out under the following reaction conditions.
* Reaction conditions
94°C for 2 minutes
↓
12 cycles of 94°C for 10 seconds → 55°C for 10 seconds → 72°C for 20 seconds as one cycle
↓
72°C for 2 minutes

Next, 5 µL of 6 × Loading Buffer Double Dye (manufactured by Nippon Gene Co., Ltd.) was added to and mixed with 25 µL of each of the PCR amplification product-containing solutions, and electrophoresis was carried out on 1.5% agarose gel. After staining with GelRed Nucleic Acid Gel Stain (manufactured by Wako Pure Chemical Industries, Ltd.) subsequent to the electrophoresis, each of the PCR amplification products was recovered using a QIAquick Gel Extraction Kit (manufactured by Qiagen AG).

### (7) Restriction enzyme treatment

To 43 µL of each of the PCR amplification product-containing solutions obtained in the section (6), 5 µL of 10 × B buffer (manufactured by Nippon Gene Co., Ltd.), 1 µL of 20 units/µL Hind III (manufactured by Nippon Gene Co., Ltd.), and 1 µL of 20 units/µL BamHI (manufactured by Nippon Gene Co., Ltd.) were mixed and reacted at 37°C for 1 hour. Similarly, distilled water was added to 500 ng of pUC19 (manufactured by Nippon Gene Co., Ltd.) to make a volume of 43 µL, and 1 µL of 20 units/µL HindIII (manufactured by Nippon Gene Co., Ltd.) and 1 µL of 20 units/µL BamHI (manufactured by Nippon Gene Co., Ltd.) were added thereto, followed by reaction at 37°C for 1 hour. Next, 250 µL of 5.5 M guanidine hydrochloride (manufactured by Wako Pure Chemical Industries, Ltd.) was added to and mixed with each of the reaction solutions which was then transferred to ECONOSPIN (manufactured by Gene Design Inc.) and centrifuged at 12000 ×g and room temperature for 1 min to remove the solution in the tube. Subsequently, 500 µL of 2 mM Tris-HCl pH 7.5 (manufactured by Nippon Gene Co., Ltd.) was added to each of the centrifuged reaction solutions which was then centrifuged at 12000 ×g and room temperature for 1 minute to remove the solution in the tube. After further centrifugation at 12000 ×g and room temperature for 1 minute, the tube was changed to a fresh one and 25 µL of 2 mM Tris-HCl pH 8.0 (manufactured by Nippon Gene Co., Ltd.) was added to each of the centrifuged reaction solutions which was then centrifuged at 12000 ×g and room temperature for 1 min, whereby each PCR amplification product and pUC19 treated with restriction enzymes (HindIII and BamHI) were recovered.

### (8) Transformation

1 µL of pUC 19 treated with a restriction enzyme and 2 µL of DNA Ligation Kit Mighty Mix (manufactured by Takara Bio Inc.) were added to and mixed with 1 µL of each of the PCR amplification product-containing solutions obtained in the section (7), followed by reaction at 16°C for 1 hour. Then, 4 µL of the reaction product in total was transformed into ECOS™ Competent *E. coli* DH5α (manufactured by Nippon Gene Co., Ltd.) which was then spread on an LB agar medium supplemented with ampicillin. Thereafter, the colonies were cultured in an LB agar medium supplemented with ampicillin at 37°C for 16 hours. Thereafter, plasmids were respectively extracted using a plasmid kit SII (manufactured by Kurabo Industries, Ltd.). Next, using the obtained plasmids, the base sequences thereof was sequenced through the sequencing service of Takara Bio Inc.

The results are shown in Fig. 4.

Fig. 4 shows whether cytosines in three sequences containing a CpG sequence (CCGG) which are confirmation regions of the methylation state are methylated cytosines or unmethylated cytosines, regarding the results of sequencing the base sequences of normal cells (hips and HDF) and cancer cells (U251-MG-P1, MCF-7, LNCaP clone FGC, and HL60). In addition, circled numbers 1 to 3 in Fig. 4 correspond to the boxed three sequences containing a CpG sequence (CCGG), shown in Fig.3, respectively.

Further, in Fig. 4, the white circle indicates that cytosine in the CpG sequence is unmethylated cytosine, and the solid circle indicates that cytosine in the CpG sequence is methylated cytosine.

As is apparent from the results in Fig. 4, it was found that cytosines in the three CpG sequence-containing sequences (CCGG), which are confirmation regions of methylation state derived from normal cells (hiPS and HDF), are unmethylated cytosines, and cytosines in the three CpG sequence-containing sequences (CCGG), which are confirmation regions of methylation state derived from cancer cells (U251-MG-P1, MCF-7, LNCaP clone FGC, and HL60), are methylated cytosines.

### Example 1 and Comparative Examples 1 to 3

In order to verify the effect of S1 nuclease in the step 1 according to the present invention, the amplification method, the methylation determination method, and the method for determining the occurrence of cancer according to the embodiment of the present invention were carried out under the following conditions.
- Example 1: In the case where a step of treating with a single strand-specific nuclease (S1 nuclease) was carried out
- Comparative Example 1: In the case where a step of treating with a single strand-specific nuclease (mung bean nuclease) was carried out
- Comparative Example 2: In the case where a step of treating with a single strand-specific nuclease (exonuclease I) was carried out
- Comparative Example 3: In the case where a step of treating with a single strand-specific nuclease was not carried out

### Example 1

### (1) Setting conditions

Samples and analysis target regions were set as follows.

### • Samples

Human induced pluripotent stem cells (hiPS): normal cells

### • Analysis target regions

A region (267 base pairs) consisting of the base sequence represented by SEQ ID NO: 1 within the promoter region of the human-derived FOXB2 gene

### (2) Extraction of genomic DNA

Genomic DNA derived from hiPS was extracted with QuickGene SP kit DNA tissue (manufactured by Kurabo Industries Co., Ltd.) according to the instructions attached.

### (3) Fragmentation treatment with restriction enzymes (BamHI and HindIII) and column purification

5 µL of 10 × B buffer (manufactured by Nippon Gene Co., Ltd.), 1 µL of 20 units/µL BamHI (manufactured by Nippon Gene Co., Ltd.), and 1 µL of 20 units/µL HindIII (manufactured by Nippon Gene Co., Ltd.) were added to 150 ng of the genomic DNA obtained in the section (2), which was then adjusted to a total volume of 50 µL with sterile water. This was followed by reaction at 37°C for 1 hour. Next, 250 µL of 5.5 M guanidine hydrochloride (manufactured by Wako Pure Chemical Industries, Ltd.) was added to and mixed therewith. The mixture was transferred to ECONOSPIN (manufactured by Gene Design Inc.), and centrifuged at 20000 ×g and room temperature for 3 minutes to remove the solution in the tube. Subsequently, 600 µL of 2 mM Tris-HCl pH 7.5 (manufactured by Nippon Gene Co., Ltd.) and 600 µL of 80% ethanol (manufactured by Wako Pure Chemical Industries, Ltd.) were added thereto, followed by centrifugation at 12000 ×g and room temperature for 1 minute to remove the solution in the tube. Furthermore, after centrifugation at 12000 ×g and room temperature for 5 minutes, the tube was changed to a fresh one, and 30 µL of 2 mM Tris-HCl pH 8.0 (manufactured by Nippon Gene Co., Ltd.) was added, followed by centrifugation at 12000 ×g and room temperature for 1 minute, whereby genomic DNA treated with restriction enzymes (BamHI and HindIII) was obtained.

### (4) Treatment with single strand-specific nuclease (S1 nuclease) and column purification

4 µL of 10 × S1 nuclease buffer (manufactured by Takara Bio Inc.) and 1 µL of 180 units/µL S1 nuclease (manufactured by Takara Bio Inc.) were added to 25 µL of a Tris-HCl solution containing the genomic DNA obtained in the section (3), which was then adjusted to a total volume of 40 µL with sterile water. This was followed by reaction at 23°C for 15 minutes, 1 µL of 0.5 M EDTA was added. Next, 200 µL of 5.5 M guanidine hydrochloride (manufactured by Wako Pure Chemical Industries, Ltd.) and 200 µL of 20 mM Tris-HCl pH 6.6 (manufactured by Wako Pure Chemical Industries, Ltd.) were added and mixed therewith. The mixture was transferred to ECONOSPIN (manufactured by Gene Design Inc.) and centrifuged at 20000 ×g and room temperature for 3 minutes to remove the solution in the tube. Next, 600 µL of 2 mM Tris-HCl pH 7.5 (manufactured by Nippon Gene Co., Ltd.) and 600 µL of 80% ethanol (manufactured by Wako Pure Chemical Industries, Ltd.) were added thereto, followed by centrifugation at 12000 ×g and room temperature for 1 minute to remove the solution in the tube. Furthermore, after centrifugation at 12000 ×g and room temperature for 5 minutes, the tube was changed to a fresh one, and 30 µL of 2 mM Tris-HCl pH 8.0 (manufactured by Nippon Gene Co., Ltd.) was added, followed by centrifugation at 12000 ×g and room temperature for 1 minute, whereby genomic DNA treated with a single strand-specific nuclease (S1 nuclease) was obtained.

### (5) Treatment with methylation-sensitive restriction enzyme (HapII) and column purification

4 µL of 10 × L buffer (manufactured by Takara Bio Inc.) and 1 µL of 25 units/µL HapII (manufactured by Takara Bio Inc.) were added to 25 µL of a Tris-HCl solution containing the genomic DNA obtained in the section (4), which was then adjusted to a total volume of 40 µL with sterile water. This was followed by reaction at 37°C for 2 hours. Next, 200 µL of 5.5 M guanidine hydrochloride (manufactured by Wako Pure Chemical Industries, Ltd.) and 200 µL of 20 mM Tris-HCl pH 6.6 (manufactured by Wako Pure Chemical Industries, Ltd.) were added and mixed therewith. The mixture was transferred to ECONOSPIN (manufactured by Gene Design Inc.) and centrifuged at 20000 ×g and room temperature for 3 minutes to remove the solution in the tube. Subsequently, 600 µL of 2 mM Tris-HCl pH 7.5 (manufactured by Nippon Gene Co., Ltd.) and 600 µL of 80% ethanol (manufactured by Wako Pure Chemical Industries, Ltd.) were added, followed by centrifugation at 12000 ×g and room temperature for 1 minute to remove the solution in the tube. Furthermore, after centrifugation at 12000 ×g and room temperature for 5 minutes, the tube was changed to a fresh one, and 30 µL of 2 mM Tris-HCl pH 8.0 (manufactured by Nippon Gene Co., Ltd.) was added, followed by centrifugation at 12000 ×g and room temperature for 1 minute, whereby genomic DNA treated with a methylation-sensitive restriction enzyme (HapII) was obtained.

The sequences recognized by HapII and HpaII are boxed three CpG sequence-containing sequences (CCGG) in the region (267 base pairs) consisting of the base sequence represented by SEQ ID NO: 1 within the promoter region of the FOXB2 gene of the human cell-derived genomic DNA shown in Fig. 3.

### (6) PCR amplification reaction

To 1 µL of a Tris-HCl solution containing the genomic DNA obtained in the section (5) were added 2.5 µL of 10 × buffer for Blend Taq (manufactured by Toyobo Co., Ltd.), 2 µL of 2 mM dNTPs (manufactured by Toyobo Co., Ltd.), 0.2 µL of 2.5 units/µL Blend Taq-Plus (manufactured by Toyobo Co., Ltd.), 1 µL of 10 µM forward primer (h-F-Fox), and 1 µL of 10 µM reverse primer (h-R-Fox), which was then adjusted to a volume of 25 µL with sterile water and used as a reaction solution for PCR.

The size (number of base pairs) of the amplification product obtained by the primers is 267 base pairs (SEQ ID NO: 1).

In addition, a reaction solution for PCR was prepared in the same manner as described above, except that the genomic DNA obtained in the section (3) was used as a template, and this was used as a control.

Each of the reaction solutions for PCR was set in a thermal cycler (manufactured by Bio-Rad Laboratories Ltd.), and a PCR amplification reaction was carried out under the following reaction conditions.
* Reaction conditions
94°C for 2 minutes
↓
50 cycles of 94°C for 4 seconds → 68°C for 15 seconds as one cycle
↓
68°C for 1 minute

### (7) Electrophoresis

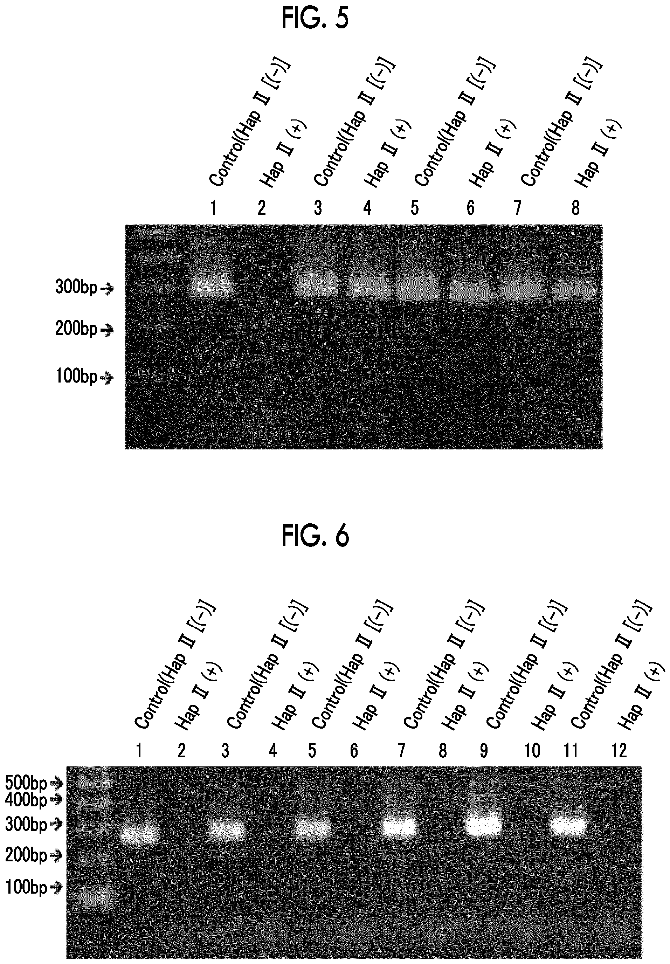

5 µL of 6 × Loading Buffer Double Dye (manufactured by Nippon Gene Co., Ltd.) was added to and mixed with 25 µL of each of solutions containing the amplification products obtained in the section (6), and the mixture was electrophoresed on a 2% agarose gel. The electrophoresis was followed by staining with a GelRed Nucleic Acid Gel Stain (manufactured by Wako Pure Chemical Industries, Ltd.), and the amplification products were respectively confirmed by a UV irradiation apparatus. The results are shown in Fig. 5 (lanes 1 and 2).

### Comparative Example 1

The amplification product was confirmed in the same manner as in Example 1, except that a mung bean nuclease (manufactured by Takara Bio Inc.) was used in place of S1 nuclease as the single strand-specific nuclease in Example 1. The results are shown in Fig. 5 (lanes 3 and 4).

### Comparative Example 2

The amplification product was confirmed in the same manner as in Example 2, except that exonuclease I (manufactured by Takara Bio Inc.) was used in place of S1 nuclease as the single strand-specific nuclease in Example 2. The results are shown in Fig. 5 (lanes 5 and 6).

### Comparative Example 3

The amplification product was confirmed in the same manner as in Example 1, except that (4) Treatment with single strand-specific nuclease in Example 1 was not carried out. The results are shown in Fig. 5 (lanes 7 and 8).

In addition, samples, single strand-specific nucleases, methylation-sensitive restriction enzymes, and the presence or absence of amplification products in individual lanes of Fig. 5 are summarized in Table 3 below.

**Table 3**

| | **Lane** | **Sample** | **single-strand-specific nuclease** | **Methylation-sensitive restriction enzyme** | **Presence or absence of amplification** |
|---|---|---|---|---|---|
| Example 1 | 1 | hiPS (normal cell) | S1 nuclease | - | Present |
| | 2 | | | HapII | Absent |
| Comparative Example 1 | 3 | | mung bean nuclease | - | Present |
| | 4 | | | HapII | Present |
| Comparative Example 2 | 5 | | Exonuclease I | - | Present |
| | 6 | | | HapII | Present |
| Comparative Example 3 | 7 | | | - | Present |
| | 8 | | | HapII | Present |

From the results in Fig. 5, in the case where S1 nuclease was used as a single strand-specific nuclease, hiPS were treated with a methylation-sensitive restriction enzyme, and a PCR amplification reaction was carried out using the primer pair according to the present invention (lane 2), it was found that a target amplification product [region (267 base pairs) consisting of the base sequence represented by SEQ ID NO: 1], that is, an analysis target region could not be obtained.

Therefore, it can be determined that the analysis target region of hiPS was cleaved with a methylation-sensitive restriction enzyme and was therefore in an unmethylated state. That is, it can be determined that hiPS derived from which the analysis target region are normal cells.

On the other hand, in the case where a mung bean nuclease was used as the single strand-specific nuclease (lane 4), in the case where exonuclease I was used as the single strand-specific nuclease (lane 6), and in the case where the step of treating with a single strand-specific nuclease was not carried out (lane 8), it was found that a target amplification product [a region (267 base pairs) consisting of the base sequence represented by SEQ ID NO: 1], that is, an amplification product having the same size as that of the analysis target region could be obtained, in the case where hiPS were treated with a methylation-sensitive restriction enzyme and a PCR amplification reaction was carried out using the primer pair according to the present invention.

However, in Comparative Examples 1 to 3, an amplification product was obtained despite the use of the same normal hiPS-derived genomic DNA as that used in Example 1 as a template, so that this amplification product was considered to be a non-specific amplification product which was produced as a result of amplification of artificially or intrinsically existing single-stranded DNA as a template.

Therefore, it was found that amplification of a non-specific amplification product which can cause false positives can be suppressed, and amplification of the methylated analysis target region, determination of methylation, and determination of cancer can be carried out with convenience and high accuracy, by treating the sample (DNA) with S1 nuclease, that is, carrying out the step 1 according to the present invention.

### Example 2: Analysis of FOXB2 gene of various normal cells and various cancer cells

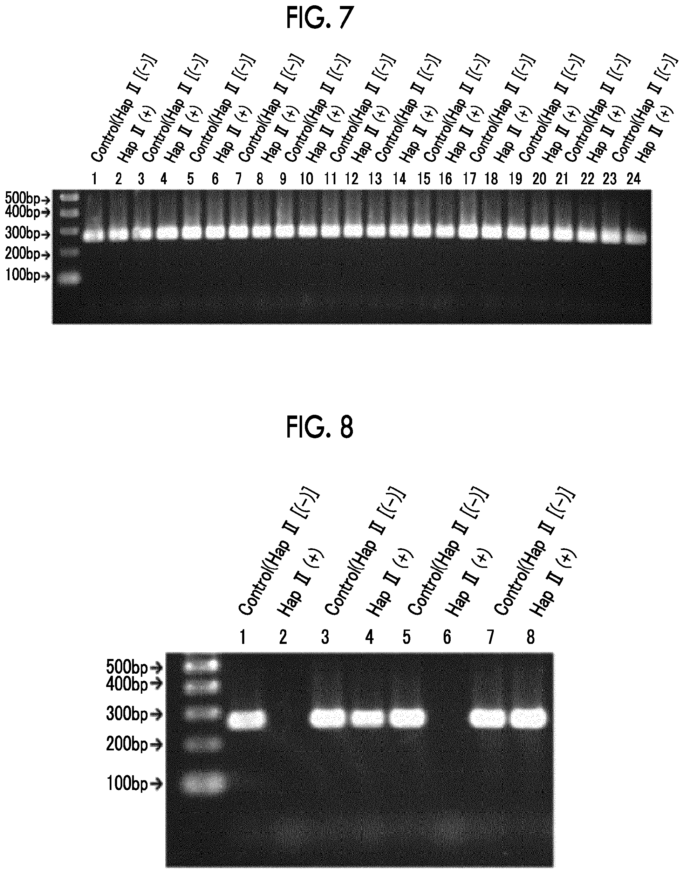

The amplification product was confirmed in the same manner as in Example 1, except that the following normal cells and cancer cells were used as samples in Example 1. The results are shown in Fig. 6 (normal cells) and Fig. 7 (cancer cells), respectively.

### • Normal cells

Normal fibroblasts (HDF)
Normal fibroblasts (CCD-8Lu)
Normal fibroblasts (WI-38)
HIV and HBV negative sera (Normal whole blood)
Induced pluripotent stem cells (hiPS)
Whole fetal cells (HE23)

### • Cancer cells

Neuroblastoma cells (IMR-32)
Glioblastoma cells (U251-MG-P1)
Uterine fibroid cells (HeLa)
Liver cancer cells (HepG2)
Colon adenocarcinoma cells (WiDr)
Non-small cell lung cancer cells (NCI-H460)
Pancreatic adenocarcinoma cells (CFPAC-1)
Ovarian teratoma cells (PA-1)
Mammary carcinoma cells (MCF-7)
Prostate cancer cells (LNCaP clone FGC)
T-lymphocytic leukemia cells (Jurkat)
Myeloid leukemia cells (HL-60)

In addition, samples, methylation-sensitive restriction enzymes, and the presence or absence of amplification products in individual lanes of Fig. 6 are summarized in Table 4 below, and samples, methylation-sensitive restriction enzymes, and the presence or absence of amplification products in individual lanes of Fig. 7 are summarized in Table 5 below.

**Table 4**

| | **Lane** | **Sample** | **Methylation-sensitive restriction enzyme** | **Presence or absence of amplification product** |
|---|---|---|---|---|
| Example 2 | 1 | HDF (normal cell) | - | Present |
| | 2 | | HapII | Absent |
| | 3 | CCD-8Lu (normal cell) | - | Present |
| | 4 | | HapII | Absent |
| | 5 | WI-38 (normal cell) | - | Present |
| | 6 | | HapII | Absent |
| | 7 | Normal whole blood (normal cell) | - | Present |
| | 8 | | HapII | Absent |
| | 9 | hiPS (normal cell) | - | Present |
| | 10 | | HapII | Absent |
| | 11 | HE23 (normal cell) | - | Present |
| | 12 | | HapII | Absent |

**Table 5**

| | **Lane** | **Sample** | **Methylation-sensitive restriction enzyme** | **Presence or absence of amplification product** |
|---|---|---|---|---|
| | 1 | IMR-32 (cancer cell) | - | Present |
| | 2 | | HapII | Present |
| | 3 | U251-MG-P1 (cancer cell) | - | Present |
| | 4 | | HapII | Present |
| | 5 | HeLa (cancer cell) | - | Present |
| | 6 | | HapII | Present |
| | 7 | HepG2 (cancer cell) | - | Present |
| | 8 | | HapII | Present |
| | 9 | WiDr (cancer cell) | - | Present |
| | 10 | | HapII | Present |
| | 11 | NCl-H460 (cancer cell) | - | Present |
| | 12 | | HapII | Present |
| Example 2 | 13 | CFPAC-1 (cancer cell) | - | Present |
| | 14 | | HapII | Present |
| | 15 | PA-1 (cancer cell) | - | Present |
| | 16 | | HapII | Present |
| | 17 | MCF-7 (cancer cell) | - | Present |
| | 18 | | HapII | Present |
| | 19 | LNCaP clone FGC (cancer cell) | - | Present |
| | 20 | | HapII | Present |
| | 21 | Jurkat (cancer cell) | - | Present |
| | 22 | | HapII | Present |
| | 23 | HL-60 (cancer cell) | - | Present |
| | 24 | | HapII | Present |

From the results in Fig. 6, in the cases of using various normal cells (lanes 2, 4, 6, 8, 10, and 12), no target amplification product [a region (267 base pairs) consisting of the base sequence represented by SEQ ID NO: 1] was confirmed in either case, indicating that no analysis target region was obtained.

That is, it can be determined that the analysis target region was cleaved by a methylation-sensitive restriction enzyme and was therefore in an unmethylated state. Therefore, it can be determined from these results that various normal cells derived from the analysis target region are normal cells.

From the results in Fig. 7, it was found that, in the cases where various cancer cells were used (lanes 2, 4, 6, 8, 10, 12, 14, 16, 18, 20, 22, and 24), the target amplification product [a region (267 base pairs) consisting of the base sequence represented by SEQ ID NO: 1] was confirmed, indicating that the analysis target region was obtained.

That is, it can be determined that the analysis target region was not cleaved by a methylation-sensitive restriction enzyme and was therefore in a methylated state. Therefore, it can be determined from these results that various cancer cells derived from the analysis target region are cancer cells.

From the above, according to the present invention, it was found that amplification and methylation determination of methylated analysis target region and determination of cancer can be carried out with convenience and high accuracy.

### Example 3: Analysis of FOXB2 gene of cancer cells derived from breast cancer patient

The amplification product was confirmed in the same manner as in Example 1, except that cancer cells derived from two breast cancer patients [breast cancer patient 1 (stage IIIA) and breast cancer patient 2 (stage II)] and normal cells in the vicinity of the cancer cells were used as samples in Example 1. The results are shown in Fig. 8.

In addition, samples, methylation-sensitive restriction enzymes, and the presence or absence of amplification products in individual lanes of Fig. 8 are summarized in Table 6 below.

**Table 6**

| | **Lane** | **Sample** | **Methylation-sensitive restriction enzyme** | **Presence or absence of amplification product** |
|---|---|---|---|---|
| Example 3 | 1 | Normal cells in vicinity of cancer cells | - | Present |
| | 2 | | HapII | Absent |
| | 3 | cancer cell | - | Present |
| | 4 | | HapII | Present |
| | 5 | Normal cells in vicinity of cancer cells | - | Present |
| | 6 | | HapII | Absent |
| | 7 | cancer cell | - | Present |
| | 8 | | HapII | Present |

From the results in Fig. 8, it was found that, in cases where cancer cells derived from breast cancer patient 1 and breast cancer patient 2 were used (lanes 4 and 8), the target amplification product [a region (267 base pairs) consisting of the base sequence represented by SEQ ID NO: 1] was confirmed, indicating that the analysis target region was obtained.

That is, it can be determined that the analysis target region was not cleaved by a methylation-sensitive restriction enzyme and was therefore in a methylated state. Therefore, it can be determined from these results that various cancer cells derived from the analysis target region are cancer cells.

On the other hand, in the cases of using various normal cells (lanes 2 and 6), no target amplification product [a region (267 base pairs) consisting of the base sequence represented by SEQ ID NO: 1] was confirmed in either case, indicating that no analysis target region was obtained.

That is, it can be determined that the analysis target region was cleaved by a methylation-sensitive restriction enzyme and was therefore in an unmethylated state. Therefore, it can be determined from these results that various normal cells in the vicinity of cancer cells derived from the analysis target region are normal cells.

From the above, it was found that amplification and methylation determination of the methylated analysis target region, and determination of cancer can be carried out even in an actual clinical specimen.

Further, it was also found that amplification and methylation determination of the methylated analysis target region, and determination of cancer can be carried out without causing false positives even in normal cells in the vicinity of cancer cells.

### Example 4: Analysis of FOXB2 gene of dog-derived cancer cells

The amplification product was confirmed in the same manner as in Example 1, except that cancer cells derived from a dog [MDCK cells (benign cancer cells having no metastatic potential, lung cancer cells, and liver cancer cells]) were used as samples in Example 1, the analysis target region was set to a region (433 base pairs) consisting of the base sequence represented by SEQ ID NO: 4 within the promoter region of the FOXB2 gene derived from a dog, and d-F-Fox and d-R-Fox were used as the primer pair according to the present invention.

The region to be amplified by the primer pair (d-F-Fox and d-R-Fox) according to the present invention is a region (433 base pairs) consisting of the base sequence represented by SEQ ID NO: 4.

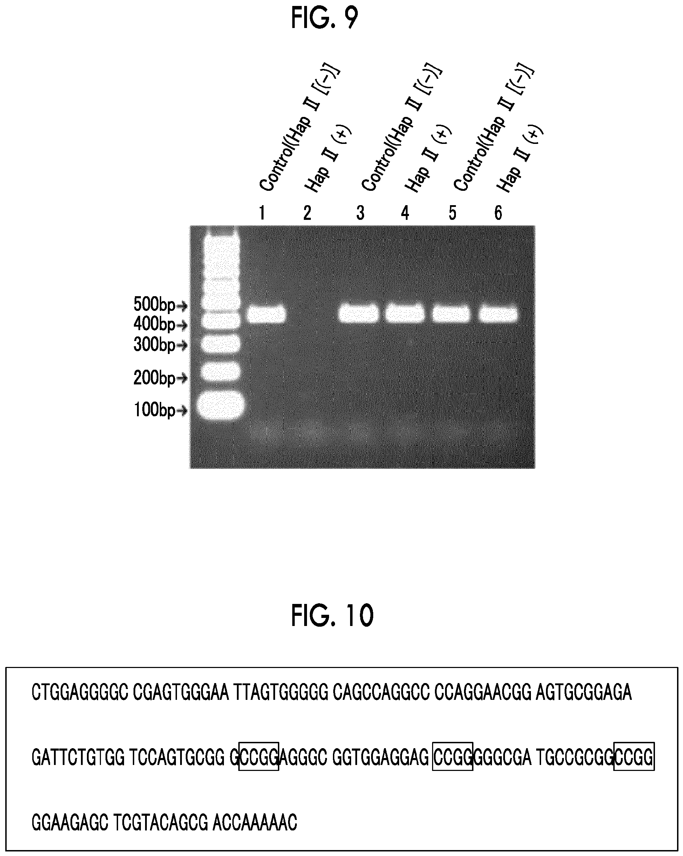

The results are shown in Fig. 9.

In addition, cells and methylation-sensitive restriction enzymes in individual lanes of Fig. 9 are summarized in Table 7 below.

**Table 7**

| | **Lane** | **Sample** | **Methylation-sensitive restriction enzyme** | **Presence or absence of amplification product** |
|---|---|---|---|---|
| Example 4 | 1 | MDCK (benign cancer cell) | - | Present |
| | 2 | | HapII | Absent |
| | 3 | Lung cancer cell (cancer cell) | - | Present |
| | 4 | | HapII | Present |
| | 5 | Liver cancer cell (cancer cell) | - | Present |
| | 6 | | HapII | Present |

From the results in Fig. 9, it was found that, in the cases where MDCK cells (benign cancer cells having no metastatic potential) were used (lane 2), no target amplification product [a region (433 base pairs) consisting of the base sequence represented by SEQ ID NO: 4] was confirmed in either case, indicating that no analysis target region was obtained.

That is, it can be determined that the analysis target region was cleaved by a methylation-sensitive restriction enzyme and was therefore in an unmethylated state. Therefore, it can be determined from these results that MDCK cells (benign cancer cells having no metastatic potential) derived from the analysis target region are normal cells.

On the other hand, it was found that, in the cases of using lung cancer cells and liver cancer cells (lanes 4 and 6), the target amplification product [a region (433 base pairs) consisting of the base sequence represented by SEQ ID NO: 1] was confirmed, indicating that the analysis target region was obtained.

That is, it can be determined that the analysis target region was not cleaved by a methylation-sensitive restriction enzyme and was therefore in a methylated state. Therefore, it can be determined from these results that lung cancer cells and liver cancer cells derived from the analysis target region are cancer cells.

From the above, according to the present invention, it was found that amplification and methylation determination of the methylated analysis target region, and determination of the occurrence of cancer can be carried out even in cells derived from a dog. In addition, it was also found that benign cancer cells having no metastatic potential can be determined as normal cells.

Further, it was suggested that the amplification method and methylation determination method of the methylated analysis target region, and the method for determining cancer according to the embodiment of the present invention can be applied to cancer screening of animals such as dogs.

### Example 5: Analysis of FOXB2 gene using plasma derived from cancer patient

As described hereinbefore, cfDNA refers to DNA derived from the death of cancer (tumor) cells, blood cells such as white blood cells, and cells or the like derived from individual tissues and is present in body fluids such as blood and urine. In addition, it has been reported that the amount of cfDNA in cancer patients is higher than that in normal subjects.

Therefore, cancer (tumor) screening (liquid biopsy) using a body fluid such as blood or urine is attracting attention instead of conventional biopsy of collecting a cancer (tumor) tissue using an endoscope or a needle.

Therefore, the present inventors have verified whether or not the method of the present invention can be applied to liquid biopsy.

### (1) Setting conditions

Samples and analysis target regions were set as follows.

### • Samples

Plasma derived from liver cancer patient
Plasma derived from lung cancer patient

### • Analysis target regions

A region (149 base pairs) consisting of the base sequence represented by SEQ ID NO: 11 within the promoter region of the human-derived FOXB2 gene

### (2) Extraction of cfDNA

Each cfDNA was extracted with NucleoSpin™ Plasma XS (manufactured by Macherey-Nagel Inc.) according to the instructions attached.

### (3) Fragmentation treatment with restriction enzyme (MboI), treatment with methylation-sensitive restriction enzyme (HpaII), and column purification

10 µL of 10 × CutSmart Buffer (manufactured by NEB Co., Ltd.), 1 µL of 25 units/µL MboI (manufactured by NEB Co., Ltd.) and 3 µL of 50 units/µL HpaII (manufactured by NEB Co., Ltd.) were added to each of the cfDNAs obtained in the section (2), which was then adjusted to a total volume of 100 µL with sterile water. This was followed by reaction at 37°C for 1 hour and 30 minutes. Next, 500 µL of 5.5 M guanidine hydrochloride (manufactured by Wako Pure Chemical Industries, Ltd.) and 500 µL of 20 mM Tris-HCl pH 7.0 (manufactured by Nippon Gene Co., Ltd.) were added and mixed therewith. Then, the mixture was transferred to ECONOSPIN (manufactured by Gene Design Inc.) and centrifuged at 13000 ×g and room temperature for 2 minutes to remove the solution in the tube. Thereafter, 600 µL of 2 mM Tris-HClpH 7.5 (manufactured by Nippon Gene Co., Ltd.) and 600 µL of 80% ethanol (manufactured by Wako Pure Chemical Industries, Ltd.) were added thereto, followed by centrifugation at 13000 ×g and room temperature for 1 minute to remove the solution in the tube. This operation was repeated once more, followed by further centrifugation at 13000 ×g and room temperature for 3 minutes, and the tube was changed to a fresh one, followed by addition of 50 µL of sterile water and centrifugation at 13000 ×g and room temperature for 1 minute to obtain cfDNAs treated with a restriction enzyme (MboI) and a methylation-sensitive restriction enzyme (HpaII).

### (4) Treatment with S1 nuclease and column purification

10 µL of 10 × S1 nuclease buffer (manufactured by Takara Bio Inc.) and 1 µL of 180 units/µL **S1** nuclease were added to each 50 µL of sterile water containing the cfDNAs obtained in the section (3), which was then adjusted to a volume of 100 µL with sterile water. This was followed by reaction at 23°C for 15 minutes. Next, 500 µL of 5.5 M guanidine hydrochloride (manufactured by Wako Pure Chemical Industries, Ltd.) and 500 µL of 20 mM Tris-HCl pH 7.0 (manufactured by Nippon Gene Co., Ltd.) were added and mixed therewith. Then, the mixture was transferred to ECONOSPIN (manufactured by Gene Design Inc.) and centrifuged at 13000 ×g and room temperature for 2 minutes to remove the solution in the tube. Thereafter, 600 µL of 2 mM Tris-HCl pH 7.5 (manufactured by Nippon Gene Co., Ltd.) and 600 µL of 80% ethanol (manufactured by Wako Pure Chemical Industries, Ltd.) were added thereto, followed by centrifugation at 13000 ×g and room temperature for 1 minute to remove the solution in the tube. This operation was repeated once more, followed by further centrifugation at 13000 ×g and room temperature for 3 minutes, and the tube is changed to a fresh one, followed by addition of 50 µL of sterile water and centrifugation at 13000 ×g and room temperature for 1 minute to obtain cfDNAs treated with S1 nuclease.

### (5) Treatment with methylation-sensitive restriction enzyme (HpaII) and column purification

10 µL of 10 × CutSmart Buffer (manufactured by NEB Co., Ltd.) and 3 µL of 50 units/µL HpaII (manufactured by NEB Co., Ltd.) were added to each 50 µL of sterile water containing the cfDNAs obtained in the section (4), which was then adjusted to a total volume of 100 µL with sterile water. This was followed by reaction at 37°C for 1 hour and 30 minutes. Next, 500 µL of 5.5 M guanidine hydrochloride (manufactured by Wako Pure Chemical Industries, Ltd.) and 500 µL of 20 mM Tris-HCl pH 7.0 (manufactured by Nippon Gene Co., Ltd.) were added and mixed therewith. Then, the mixture was transferred to ECONOSPIN (manufactured by Gene Design Inc.) and centrifuged at 13000 ×g and room temperature for 2 minutes to remove the solution in the tube. Thereafter, 600 µL of 2 mM Tris-HCl pH 7.5 (manufactured by Nippon Gene Co., Ltd.) and 600 µL of 80% ethanol (manufactured by Wako Pure Chemical Industries, Ltd.) were added thereto, followed by centrifugation at 13000 ×g and room temperature for 1 minute to remove the solution in the tube. This operation was repeated once more, followed by further centrifugation at 13000 ×g and room temperature for 3 minutes, and the tube was changed to a fresh one, followed by addition of 50 µL of sterile water and centrifugation at 13000 ×g and room temperature for 1 minute to obtain cfDNAs treated with a methylation-sensitive restriction enzyme (HpaII).

The sequences recognized by HpaII and HapII are boxed three CpG sequence-containing sequences (CCGG) in the region (149 base pairs) consisting of the base sequence represented by SEQ ID NO: 11 within the promoter region of the human-derived FOXB2 gene shown in Fig. 10.

### (6) Amplification reaction by digital PCR method

To each 3 µL of sterile water containing the cfDNAs obtained in the section (5) were added 11 µL of ddPCR™ Supermix for Probes (manufactured by Bio-Rad Laboratories Ltd.), 0.8 µL of 50 units/µL HapII (manufactured by Takara Bio Inc.), 1 µL of 20 µM forward primer (Hcf-F-fox), 1 µL of 20 µM reverse primer (hcf-R-fox), and 1 µL of 10 µM probe [hcf-Probe-fox (labeled with FAM at 5' end and BHQ1 at 3' end)], which was then adjusted to a total volume of 22 µL with sterile water. The resulting solutions were used as reaction solutions for digital PCR. Thereafter, each of the reaction solutions was set in a thermal cycler (manufactured by Bio-Rad Laboratories Ltd.) and reacted at 37°C for 40 minutes. Subsequently, droplets were prepared by Automated Droplet Generator, and the droplets were dispensed in 96-well plates. Then, an amplification reaction by the digital PCR method was carried out in the thermal cycler (manufactured by Bio-Rad Laboratories Ltd.) under the following reaction conditions.
* Reaction conditions
95°C for 10 minutes
↓
40 cycles of 94°C for 30 seconds → 62°C for 1 minute as one cycle
↓
98°C for 10 minutes
In addition, a GAPDH gene was selected as an endogenous control, and analysis was also carried out for a specific region of the GAPDH gene [region (149 base pairs) consisting of the base sequence represented by SEQ ID NO: 15] using the same sample (specimen). The base sequences of a forward primer, a reverse primer, and a probe used for analysis of the GAPDH gene are as follows.

The other experimental conditions are the same as described above, except that HapII was not added to the reaction solution for digital PCR.
Forward primer: gaagcttgtcatcaatggaaatccc (SEQ ID NO: 16)
Reverse primer: gggacaggaccatattgagggacac (SEQ ID NO: 17)
Probe (labeled with FAM at 5' end and BHQ1 at 3' end): caactaggatggtgtggctcccttg (SEQ ID NO: 18)

### (7) Confirmation of amplification product by fluorescence reader

Amplification products were confirmed by detecting the fluorescence from each droplet containing the amplification product amplified in the section (6) with a QX200 Droplet Reader (manufactured by Bio-Rad Laboratories Ltd.).

Analysis on the GAPDH gene was carried out once per sample (specimen) using one sample (specimen). In addition, one sample (specimen) was used for the analysis on the FOXB2 gene, and the analysis was carried out twice per sample (specimen).

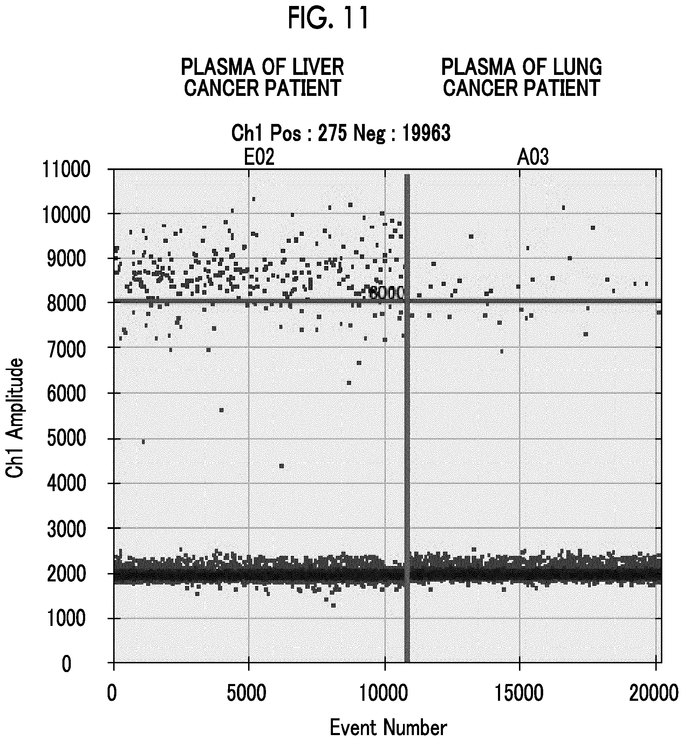

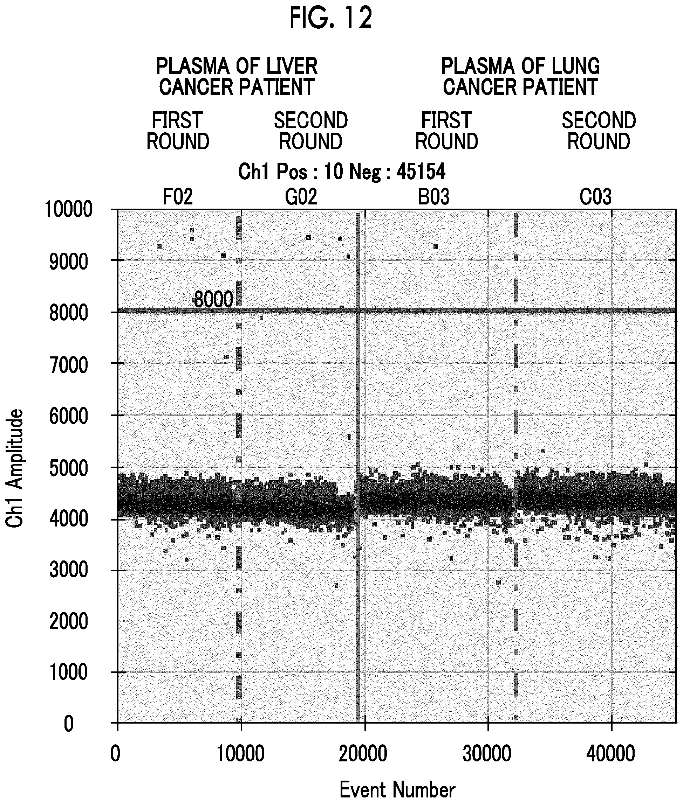

The analysis results of the GAPDH gene are shown in Fig. 11, and the analysis results of the FOXB2 gene are shown in Fig. 12.

With respect to the GAPDH gene, the threshold value as a standard for determination of positive or negative was set to a fluorescence intensity of 8000 with reference to the amplification results of the GAPDH gene. In addition, with respect to the FOXB2 gene, the threshold value was set to a fluorescence intensity of 8000 with reference to the amplification results of the FOXB2 gene. That is, in the case where there was at least one droplet having a fluorescence intensity exceeding 8000, it was determined to be positive (a target amplification product was obtained), and in the case where there was no droplet having a fluorescence intensity exceeding 8000, it was determined to be negative (a target amplification product was not obtained).

In addition, samples, analysis target regions, the number of positive droplets, the number of negative droplets, the total number of droplets, and the threshold values in Figs. 11 and 12 are summarized in Tables 8 and 9, respectively.

**Table 8**

| | Sample | Analysis target region | Number of positive droplets | Number of negative droplets | Total number of droplets | Threshold value |
|---|---|---|---|---|---|---|
| Example 5 | Plasma derived from liver cancer patient | Specific region ofGAPDH gene (SEQ ID NO: 15) | 253 | 10649 | 10902 | 8000 |
| | Plasma derived from lung cancer patient | | 22 | 9314 | 9336 | 8000 |

**Table 9**

| | Sample | | Analysis target region | Number of positive droplets | Number of negative droplets | Total number of droplets | Threshold value |
|---|---|---|---|---|---|---|---|
| Example 5 | Plasma derived from liver cancer patient | first round | Specific region of FOXB2 gene (SEQ ID NO: 11) | 5 | 9354 | 9359 | 8000 |
| | | second round | | 4 | 10007 | 10011 | 8000 |
| | Plasma derived from lung cancer patient | first round | | 1 | 12743 | 12744 | 8000 |
| | | second round | | 0 | 13050 | 13050 | 8000 |

From the results in Fig. 11 and Table 8, in the case where plasma derived from a liver cancer patient was used, positive droplets that emit fluorescence exceeding a threshold value could be confirmed (253 droplets), indicating that the target amplification product [specific region of GAPDH gene: region (149 base pairs) consisting of the base sequence represented by SEQ ID NO: 15] was amplified.

In addition, also in the case where plasma derived from a lung cancer patient was used, positive droplets that emit fluorescence exceeding a threshold value could be confirmed (22 droplets), indicating that the target amplification product [specific region of GAPDH gene: region (149 base pairs) consisting of the base sequence represented by SEQ ID NO: 15] was amplified.

From the above, it was shown that cfDNA is present in the samples (plasma derived from a liver cancer patient and plasma derived from a lung cancer patient).

From the results in Fig. 12 and Table 9, in the case where plasma derived from a liver cancer patient was used, positive droplets that emit fluorescence exceeding a threshold value could be confirmed (first round: 5 droplets, second round: 4 droplets), indicating that the target amplification product [specific region of FOXB2 gene: region (149 base pairs) consisting of the base sequence represented by SEQ ID NO: 11] was obtained.

In addition, also in the case where plasma derived from a lung cancer patient was used, positive droplets that emit fluorescence exceeding a threshold value could be confirmed (first round: 1 droplet), indicating that the target amplification product [specific region of FOXB2 gene: region (149 base pairs) consisting of the base sequence represented by SEQ ID NO: 11] was obtained.

That is, it can be determined that the analysis target region was not cleaved by a methylation-sensitive restriction enzyme and was therefore in a methylated state. In addition, it can be determined that a subject derived from the plasma (liver cancer patient and lung cancer patient) is affected by cancer or that cancer cells are present in the subject (liver cancer patient and lung cancer patient).

From the above, it was found that amplification of the methylated analysis target region, determination of methylation and determination of cancer using the amplification of methylated analysis target region could be carried out even in the case where plasma derived from a cancer patient was used as a sample.

Therefore, it was suggested that the amplification method and methylation determination method of the methylated analysis target region, and the method for determining cancer according to the embodiment of the present invention can be applied to liquid biopsy.

### Example 6: Analysis of FOXB2 gene using plasma derived from normal subject (simultaneous amplification/detection of FOXB2 gene and GAPDH gene)

In the case of assuming an actual clinical setting, it is preferable to simultaneously amplify and detect a target gene and a gene selected as an endogenous control from the viewpoint of the rapidity of a test.

Therefore, the present inventors have verified whether or not the method of the present invention can be applied to simultaneous amplification/detection of a target gene and a gene selected as an endogenous control.

The amplification product was confirmed in the same manner as in Example 5, except that plasma derived from a normal subject was used as the sample in Example 5, the threshold value was set as follows, and the following reaction solution for digital PCR was used. Experiments were carried out four times for each sample (specimen) using four samples (specimens).

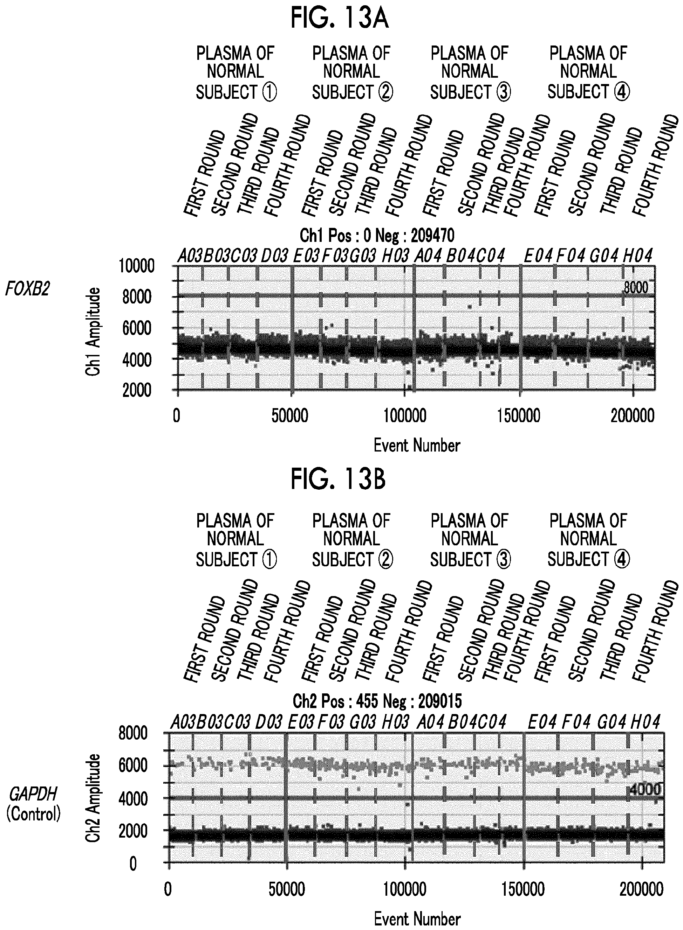

The results are shown in Fig. 13 (A and B).

### • Threshold value

With respect to the FOXB2 gene, the threshold value was set to a fluorescence intensity of 8000 with reference to the amplification results of the FOXB2 gene. In addition, with respect to the GAPDH gene, the threshold value was set to a fluorescence intensity of 4000 with reference to the amplification results of the GAPDH gene.

### • Reaction solution for digital PCR

To 3 µL of cfDNA treated with a methylation-sensitive restriction enzyme were added 1 µL of 20 µM forward primer (hcf-F-fox), 1 µL of 20 µM reverse primer (hcf-R-fox), 0.6 µL of 10 µM probe [hcf-Probe-fox (labeled with FAM at 5' end and BHQ1 at 3' end)], 1 µL of 20 µM forward primer (base sequence represented by SEQ ID NO: 16), 1 µL of 20 µM reverse primer (base sequence represented by SEQ ID NO: 17), 0.6 µL of 10 µM probe [base sequence represented by SEQ ID NO: 18 (labeled with HEX at 5' end and BHQ1 at 3' end)], 11 µL of ddPCR™ Supermix for Probes (manufactured by Bio-Rad Laboratories Ltd.), and 0.6 µL of 50 units/µL HpaII (manufactured by NEB Co., Ltd.), which was then adjusted to a total volume of 22 µL with sterile water.

In addition, samples, analysis target regions, the number of positive droplets, the number of negative droplets, the total number of droplets, and the threshold values in Figs. 13A and 13B are summarized in Tables 10 and 11, respectively.

**Table 10**

| | Sample | | Analysis target region | Number of positive droplets | Number of negative droplets | Total number of droplets | Threshold value |
|---|---|---|---|---|---|---|---|
| | Plasma derived from normal subject ① | first round | | 0 | 11846 | 11846 | 8000 |
| | | second round | | 0 | 11521 | 11521 | 8000 |
| | | third round | | 0 | 11729 | 11729 | 8000 |
| | | fourth round | | 0 | 15500 | 15500 | 8000 |
| | Plasma derived from normal subject ② | first round | | 0 | 12451 | 12451 | 8000 |
| | | second round | | 0 | 13028 | 13028 | 8000 |
| | | third round | | 0 | 12948 | 12948 | 8000 |
| Example 6 | | fourth round | Specific region of FOXB2 gene (SEQ ID NO: 11) | 0 | 14901 | 14901 | 8000 |
| | Plasma derived from normal subject ③ | first round | | 0 | 12889 | 12889 | 8000 |
| | | second round | | 0 | 15113 | 15113 | 8000 |
| | | third round | | 0 | 9359 | 9359 | 8000 |
| | | fourth round | | 0 | 10152 | 10152 | 8000 |
| | Plasma derived from normal subject ④ | first round | | 0 | 13720 | 13720 | 8000 |
| | | second round | | 0 | 15562 | 15562 | 8000 |
| | | third round | | 0 | 13624 | 13624 | 8000 |
| | | fourth round | | 0 | 15127 | 15127 | 8000 |

**Table 11**

| | Sample | | Analysis target region | Number of positive droplets | Number of negative droplets | Total number of droplets | Threshold value |
|---|---|---|---|---|---|---|---|
| | Plasma derived from normal subject ① | first round | | 15 | 11831 | 11846 | 4000 |
| | | second round | | 14 | 11507 | 11521 | 4000 |
| | | third round | | 12 | 11717 | 11729 | 4000 |
| | | fourth round | | 27 | 15473 | 15500 | 4000 |
| | Plasma derived from normal subject ② | first round | | 39 | 12412 | 12451 | 4000 |
| | | second round | | 39 | 12989 | 13028 | 4000 |
| | | third round | | 44 | 12904 | 12948 | 4000 |
| Example 6 | | fourth round | Specific region of GAPDH gene (SEQ ID NO: 15) | 36 | 14865 | 14901 | 4000 |
| | Plasma derived from normal subject ③ | first round | | 22 | 12867 | 12889 | 4000 |
| | | second round | | 22 | 15091 | 15113 | 4000 |
| | | third round | | 20 | 9339 | 9359 | 4000 |
| | | fourth round | | 17 | 10135 | 10152 | 4000 |
| | Plasma derived from normal subject ④ | first round | | 38 | 13682 | 13720 | 4000 |
| | | second round | | 41 | 15521 | 15562 | 4000 |
| | | third round | | 39 | 13585 | 13624 | 4000 |
| | | fourth round | | 30 | 15097 | 15127 | 4000 |

From the results in Fig. 13A and Table 10, in the case where plasma derived from a normal subject was used, positive droplets that emit fluorescence exceeding a threshold value could not be confirmed in all 4 specimens (samples), indicating that the target amplification product [specific region of FOXB2 gene: region (149 base pairs) consisting of the base sequence represented by SEQ ID NO: 11] was not amplified.

From the results in Fig. 13B and Table 11, in the case where plasma derived from a normal subject was used, positive droplets that emit fluorescence exceeding a threshold value could be confirmed in all 4 specimens (samples), indicating that the target amplification product [specific region of GAPDH gene: region (149 base pairs) consisting of the base sequence represented by SEQ ID NO: 15] was amplified.

That is, it can be determined that the analysis target region was cleaved by a methylation-sensitive restriction enzyme and was therefore in an unmethylated state. In addition, it can be determined that a subject derived from the plasma (normal subject) is not affected by cancer or that cancer cells are not present in the subject (normal subject).

### Example 7: Analysis of FOXB2 gene using plasma derived from various cancer patients (simultaneous amplification/detection of FOXB2 gene and GAPDH gene)

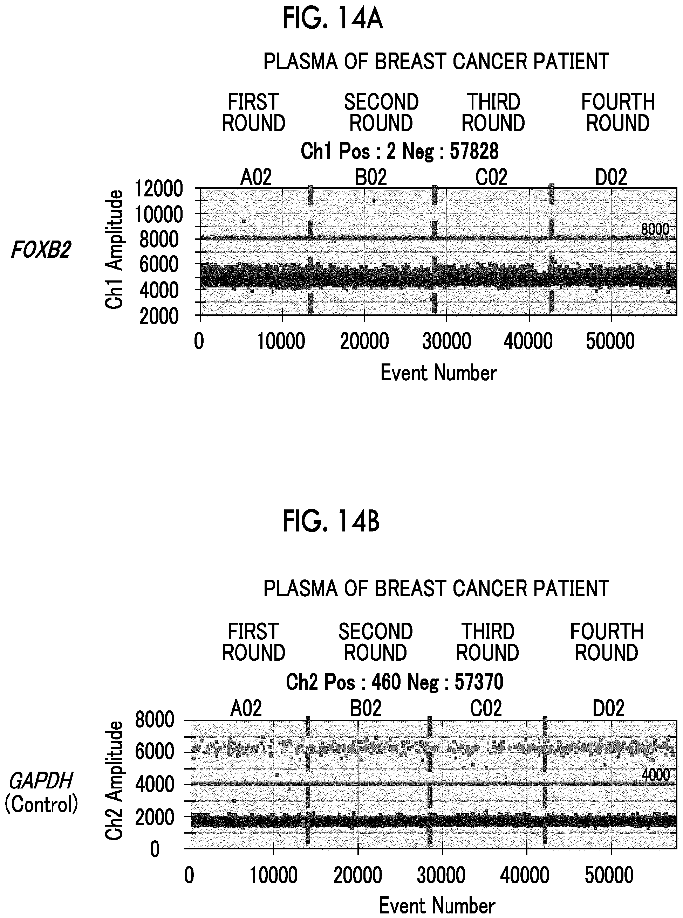

The amplification product was confirmed in the same manner **as in** Example 6, except that the following samples were used as the sample in Example 6, and the threshold value was set as follows. Experiments were carried out four times for each sample (specimen) using one sample (specimen). The results are shown in Figs. 14A to 18B, respectively.

### • Threshold value

With respect to the FOXB2 gene, the threshold value was set to a fluorescence intensity of 8000 with reference to the amplification results of the FOXB2 gene. In addition, with respect to the GAPDH gene, the threshold value was set to a fluorescence intensity of 4000 with reference to the amplification results of the GAPDH gene.

### • Samples

Plasma derived from breast cancer patient
Plasma derived from colon cancer patient
Plasma derived from pancreatic cancer patient
Plasma derived from gastric cancer patient
Plasma derived from lung cancer patient after administration of anticancer agent

In addition, samples, analysis target regions, the number of positive droplets, the number of negative droplets, the total number of droplets, and the threshold values in Figs. 14A to 18B are summarized in Tables 12 to 16, respectively.

**Table 12**

| | Sample | | Analysis target region | Number of positive droplets | Number of negative droplets | Total number of droplets | Threshold value |
|---|---|---|---|---|---|---|---|
| Example 7 | Plasma derived from breast cancer patient | first round | Specific region ofFOXB2 gene (SEQ ID NO: 11) | 1 | 13560 | 13561 | 8000 |
| | | second round | | 1 | 14940 | 14941 | 8000 |
| | | third round | | 0 | 13845 | 13845 | 8000 |
| | | fourth round | | 0 | 15483 | 15483 | 8000 |
| | | first round | Specific region ofGAPDH gene (SEQ ID NO: 15) | 91 | 13470 | 13561 | 4000 |
| | | second round | | 129 | 14812 | 14941 | 4000 |
| | | third round | | 93 | 13752 | 13845 | 4000 |
| | | fourth round | | 147 | 15336 | 15483 | 4000 |

**Table 13**

| | Sample | | Analysis target region | Number of positive droplets | Number of negative droplets | Total number of droplets | Threshold value |
|---|---|---|---|---|---|---|---|
| Example 7 | Plasma derived from colon cancer patient | first round | Specific region ofFOXB2 gene (SEQ ID NO: 11) | 10 | 14937 | 14947 | 8000 |
| | | second round | | 9 | 17749 | 17758 | 8000 |
| | | third round | | 2 | 15924 | 15926 | 8000 |
| | | fourth round | | 10 | 15046 | 15056 | 8000 |
| | | first round | Specific region ofGAPDH gene (SEQ ID NO: 15) | 94 | 14853 | 14947 | 4000 |
| | | second round | | 126 | 17632 | 17758 | 4000 |
| | | third round | | 93 | 15833 | 15926 | 4000 |
| | | fourth round | | 74 | 14982 | 15056 | 4000 |

**Table 14**

| | Sample | | Analysis target region | Number of positive droplets | Number of negative droplets | Total number of droplets | Threshold value |
|---|---|---|---|---|---|---|---|
| Example 7 | Plasma derived from pancreatic cancer patient | first round | Specific region ofFOXB2 gene (SEQ ID NO: 11) | 0 | 16567 | 16567 | 8000 |
| | | second round | | 1 | 16875 | 16876 | 8000 |
| | | third round | | 2 | 16252 | 16254 | 8000 |
| | | fourth round | | 1 | 17333 | 17334 | 8000 |
| | | first round | Specific region ofGAPDH gene (EQ ID NO: 15) | 65 | 16502 | 16567 | 4000 |
| | | second round | | 76 | 16800 | 16876 | 4000 |
| | | third round | | 81 | 16173 | 16254 | 4000 |
| | | fourth round | | 86 | 17248 | 17334 | 4000 |

**Table 15**

| | Sample | | Analysis target region | Number of positive droplets | Number of negative droplets | Total number of droplets | Threshold value |
|---|---|---|---|---|---|---|---|
| Example 7 | Plasma derived from gastric cancer patient | first round | Specific region ofFOXB2 gene (SEQ ID NO: 11) | 10 | 11088 | 11098 | 8000 |
| | | second round | | 9 | 14484 | 14493 | 8000 |
| | | third round | | 18 | 15508 | 15526 | 8000 |
| | | fourth round | | 13 | 14344 | 14357 | 8000 |
| | | first round | Specific region ofGAPDH gene (SEQ ID NO: 15) | 97 | 11001 | 11098 | 4000 |
| | | second round | | 101 | 14392 | 14493 | 4000 |
| | | third round | | 112 | 15414 | 15526 | 4000 |
| | | fourth round | | 139 | 14218 | 14357 | 4000 |

**Table 16**

| | Sample | | Analysis target region | Number of positive droplets | Number of negative droplets | Total number of droplets | Threshold value |
|---|---|---|---|---|---|---|---|
| Example 7 | Plasma derived from lung cancer patient after administration of anticancer agent | first round | Specific region of FOXB2 gene (SEQ ID NO: 11) | 0 | 13059 | 13059 | 8000 |
| | | second round | | 1 | 14076 | 14077 | 8000 |
| | | third round | | 1 | 12220 | 12221 | 8000 |
| | | fourth round | | 0 | 13952 | 13952 | 8000 |
| | | first round | Specific region of GAPDH gene (SEQ ID NO: 15) | 85 | 12974 | 13059 | 4000 |
| | | second round | | 82 | 13995 | 14077 | 4000 |
| | | third round | | 62 | 12159 | 12221 | 4000 |
| | | fourth round | | 92 | 13860 | 13952 | 4000 |

From the results in Fig. 14A and Table 12, in the case where plasma derived from a breast cancer patient was used, positive droplets that emit fluorescence exceeding a threshold value could be confirmed (first round: 1 droplet, second round: 1 droplet), indicating that the target amplification product [specific region of FOXB2 gene: region (149 base pairs) consisting of the base sequence represented by SEQ ID NO: 11] was obtained.

In addition, from the results in Fig. 14B and Table 12, in the case where plasma derived from a breast cancer patient was used, positive droplets that emit fluorescence exceeding a threshold value could be confirmed (first round: 91 droplets, second round: 129 droplets, third round: 93 droplets, fourth round: 147 droplets), indicating that the target amplification product [specific region of GAPDH gene: region (149 base pairs) consisting of the base sequence represented by SEQ ID NO: 15] was obtained.

That is, it can be determined that the analysis target region was not cleaved by a methylation-sensitive restriction enzyme and was therefore in a methylated state. In addition, it can be determined that a subject derived from the plasma (breast cancer patient) is affected by cancer or that cancer cells are present in the subject (breast cancer patient).

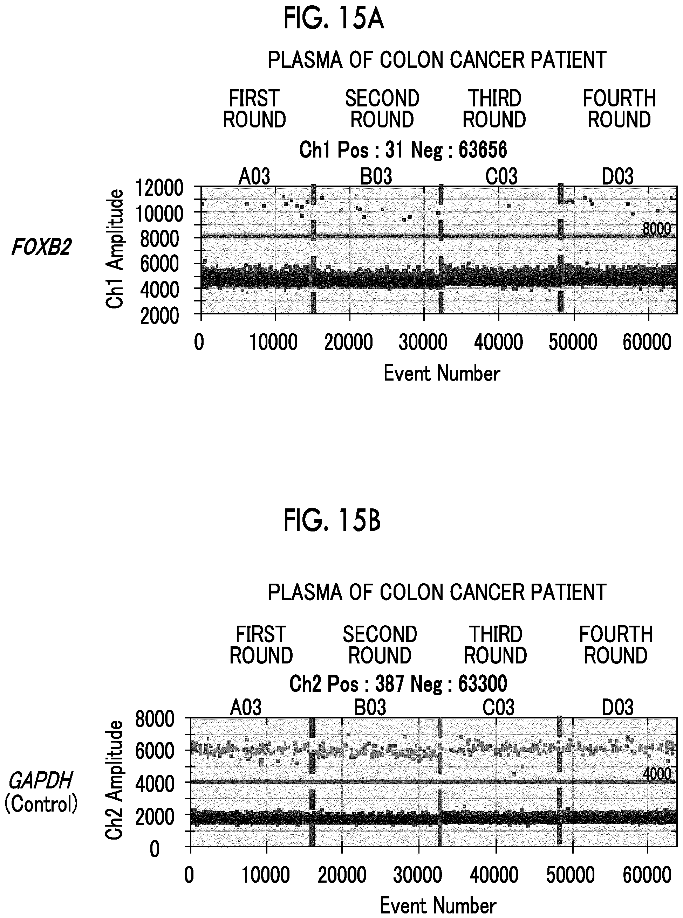

From the results in Fig. 15A and Table 13, in the case where plasma derived from a colon cancer patient was used, positive droplets that emit fluorescence exceeding a threshold value could be confirmed (first round: 10 droplets, second round: 9 droplets, third round: 2 droplets, fourth round: 10 droplets), indicating that the target amplification product [specific region of FOXB2 gene: region (149 base pairs) consisting of the base sequence represented by SEQ ID NO: 11] was obtained.

In addition, from the results in Fig. 15B and Table 13, in the case where plasma derived from a colon cancer patient was used, positive droplets that emit fluorescence exceeding a threshold value could be confirmed (first round: 94 droplets, second round: 126 droplets, third round: 93 droplets, fourth round: 74 droplets), indicating that the target amplification product [specific region of GAPDH gene: region (149 base pairs) consisting of the base sequence represented by SEQ ID NO: 15] was obtained.

That is, it can be determined that the analysis target region was not cleaved by a methylation-sensitive restriction enzyme and was therefore in a methylated state. In addition, it can be determined that a subject derived from the plasma (colon cancer patient) is affected by cancer or that cancer cells are present in the subject (colon cancer patient).

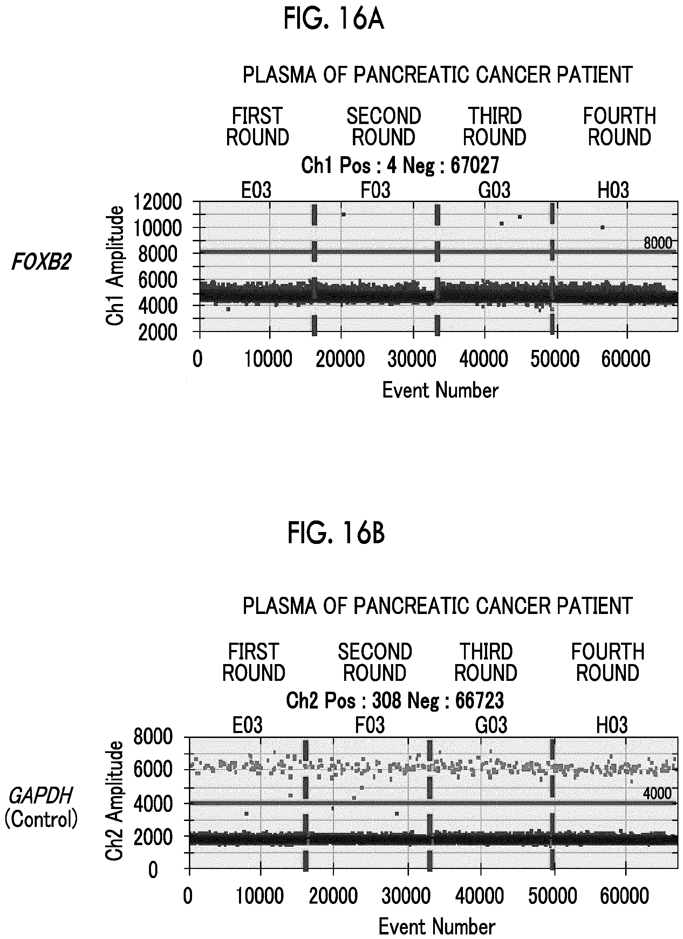

From the results in Fig. 16A and Table 14, in the case where plasma derived from a pancreatic cancer patient was used, positive droplets that emit fluorescence exceeding a threshold value could be confirmed (second round: 1 droplet, third round: 2 droplets, fourth round: 1 droplet), indicating that the target amplification product [specific region of FOXB2 gene: region (149 base pairs) consisting of the base sequence represented by SEQ ID NO: 11] was obtained.

In addition, from the results in Fig. 16B and Table 14, in the case where plasma derived from a pancreatic cancer patient was used, positive droplets that emit fluorescence exceeding a threshold value could be confirmed (first round: 65 droplets, second round: 76 droplets, third round: 81 droplets, fourth round: 86 droplets), indicating that the target amplification product [specific region of GAPDH gene: region (149 base pairs) consisting of the base sequence represented by SEQ ID NO: 15] was obtained.

That is, it can be determined that the analysis target region was not cleaved by a methylation-sensitive restriction enzyme and was therefore in a methylated state. In addition, it can be determined that a subject derived from the plasma (pancreatic cancer patient) is affected by cancer or that cancer cells are present in the subject (pancreatic cancer patient).

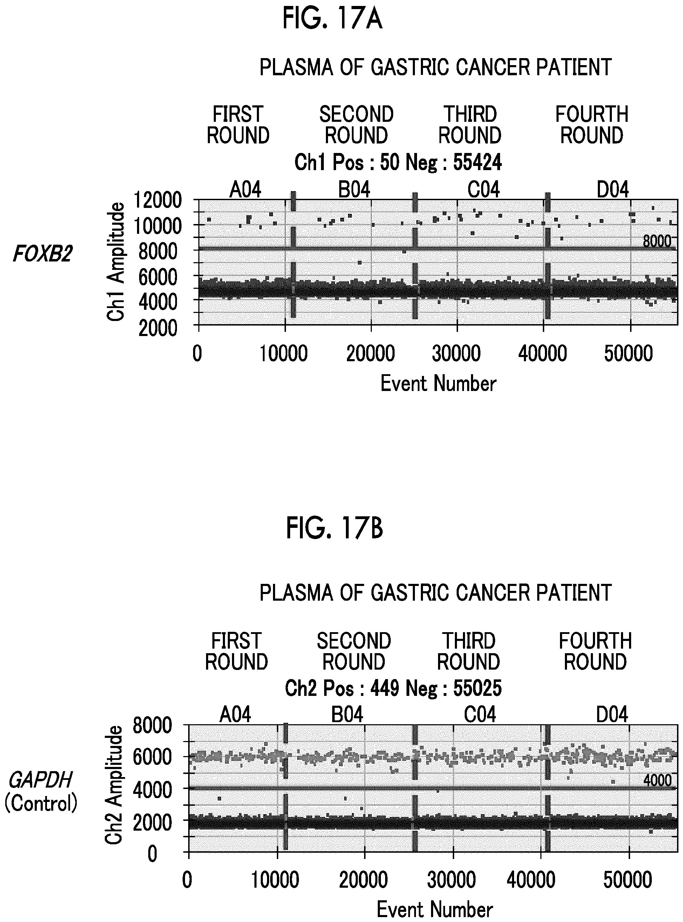

From the results in Fig. 17A and Table 15, in the case where plasma derived from a gastric cancer patient was used, positive droplets that emit fluorescence exceeding a threshold value could be confirmed (first round: 10 droplets, second round: 9 droplets, third round: 18 droplets, fourth round: 13 droplets), indicating that the target amplification product [specific region of FOXB2 gene: region (149 base pairs) consisting of the base sequence represented by SEQ ID NO: 11] was obtained.

In addition, from the results in Fig. 17B and Table 15, in the case where plasma derived from a gastric cancer patient was used, positive droplets that emit fluorescence exceeding a threshold value could be confirmed (first round: 97 droplets, second round: 101 droplets, third round: 112 droplets, fourth round: 139 droplets), indicating that the target amplification product [specific region of GAPDH gene: region (149 base pairs) consisting of the base sequence represented by SEQ ID NO: 15] was obtained.

That is, it can be determined that the analysis target region was not cleaved by a methylation-sensitive restriction enzyme and was therefore in a methylated state. In addition, it can be determined that a subject derived from the plasma (gastric cancer patient) is affected by cancer or that cancer cells are present in the subject (gastric cancer patient).

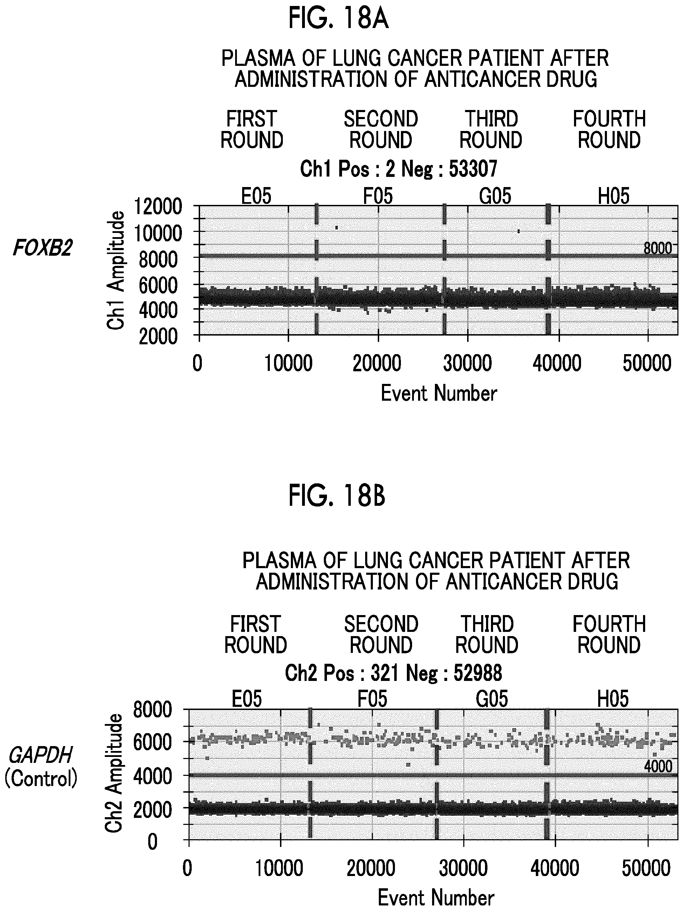

From the results in Fig. 18A and Table 16, in the case where plasma derived from a lung cancer patient after administration of an anticancer agent was used, positive droplets that emit fluorescence exceeding a threshold value could be confirmed (second round: 1 droplet, third round: 1 droplet), indicating that the target amplification product [specific region of FOXB2 gene: region (149 base pairs) consisting of the base sequence represented by SEQ ID NO: 11] was obtained.

In addition, from the results in Fig. 18B and Table 16, in the case where plasma derived from a lung cancer patient after administration of an anticancer agent was used, positive droplets that emit fluorescence exceeding a threshold value could be confirmed (first round: 85 droplets, second round: 82 droplets, third round: 62 droplets, fourth round: 92 droplets), indicating that the target amplification product [specific region of GAPDH gene: region (149 base pairs) consisting of the base sequence represented by SEQ ID NO: 15] was obtained.

That is, it can be determined that the analysis target region was not cleaved by a methylation-sensitive restriction enzyme and was therefore in a methylated state. In addition, it can be determined that a subject derived from the plasma (lung cancer patient after administration of an anticancer agent) is affected by cancer or that cancer cells are present in the subject (lung cancer patient after administration of an anticancer agent).

From Examples 6 and 7, according to the method of the present invention, amplification and methylation determination of a methylated analysis target region and determination of cancer can be carried out even in the case of simultaneous amplification/detection of a target gene and a gene selected as an endogenous control.

Therefore, it was highly suggested that the method of the present invention can be applied in an actual clinical setting.

In addition, from Figs. 18A and 18B of Example 8, it was suggested that the present invention could be used for follow-up observation of anticancer agent administration, radiation irradiation, surgical prognosis, and the like, since the target amplification product was obtained even in the case where plasma derived from a cancer patient after administration of an anticancer agent was used.

### Industrial Applicability

According to the amplification method, the methylation determination method, the method for determining cancer, and the method for obtaining data for determining cancer according to the embodiment of the present invention, amplification of methylated DNA, determination of methylation of DNA, determination cancer, and acquisition of data for determining cancer can be carried out with convenience and high accuracy in a short period of time.

### (Sequence Listing Free Text)

Spec 2055PCT_ST250002.txt

## Claims

1. A method for amplifying a methylated analysis target region in double-stranded DNA, comprising the following steps 1 and 2:
(1) a step 1 of treating double-stranded DNA containing an analysis target region with S1 nuclease and a methylation-sensitive restriction enzyme; and
(2) a step 2 of amplifying the analysis target region of the double-stranded DNA treated in the step 1.

2. The amplification method according to claim 1, wherein, in the step 1, treatment with S1 nuclease is carried out and then treatment with a methylation-sensitive restriction enzyme is carried out.

3. The amplification method according to claim 1, wherein, in the step 1, treatment with a methylation-sensitive restriction enzyme is carried out and then treatment with S1 nuclease is carried out.

4. The amplification method according to claim 1, further comprising:
a fragmentation step of treating double-stranded DNA with a restriction enzyme whose recognition sequence is not present in the analysis target region, prior to the step 1.

5. A method for determining the methylation of an analysis target region in double-stranded DNA, comprising the following steps 1 to 4:
(1) a step 1 of treating double-stranded DNA containing the analysis target region with S1 nuclease and a methylation-sensitive restriction enzyme;
(2) a step 2 of amplifying the analysis target region of the double-stranded DNA treated in the step 1;
(3) a step 3 of confirming the presence or absence of an amplification product obtained in the step 2; and
(4) a step 4 of determining whether or not the analysis target region is methylated based on the results of the step 3.

6. The methylation determination method according to claim 5, wherein, in the step 1, treatment with S1 nuclease is carried out and then treatment with a methylation-sensitive restriction enzyme is carried out.

7. The methylation determination method according to claim 5, wherein, in the step 1, treatment with a methylation-sensitive restriction enzyme is carried out and then treatment with S1 nuclease is carried out.

8. The methylation determination method according to claim 5, further comprising:
a fragmentation step of treating double-stranded DNA with a restriction enzyme whose recognition sequence is not present in the analysis target region, prior to the step 1.

9. A reagent for determining methylation of DNA, comprising:
S1 nuclease; and
a methylation-sensitive restriction enzyme.

10. A method for determining cancer, comprising the following steps 1 to 4:
(1) a step 1 of treating double-stranded DNA containing an analysis target region with S1 nuclease and a methylation-sensitive restriction enzyme;
(2) a step 2 of amplifying the analysis target region of the double-stranded DNA treated in the step 1;
(3) a step 3 of confirming the presence or absence of an amplification product obtained in the step 2; and
(4) a step 4 of determining cancer based on the results of the step 3.

11. The cancer determination method according to claim 10, wherein, in the step 1, treatment with S1 nuclease is carried out and then treatment with a methylation-sensitive restriction enzyme is carried out.

12. The cancer determination method according to claim 10, wherein, in the step 1, treatment with a methylation-sensitive restriction enzyme is carried out and then treatment with S1 nuclease is carried out.

13. The cancer determination method according to claim 10, further comprising:
a fragmentation step of treating DNA with a restriction enzyme whose recognition sequence is not present in an analysis target region, prior to the step 1.

14. The cancer determination method according to claim 10, wherein the analysis target region is a base sequence containing a base sequence represented by SEQ ID NO: 1, SEQ ID NO: 4, or SEQ ID NO: 11.

15. A method for obtaining data for determining cancer, comprising the following steps 1 to 3:
(1) a step 1 of treating double-stranded DNA containing an analysis target region with S1 nuclease and a methylation-sensitive restriction enzyme;
(2) a step 2 of amplifying the analysis target region of the double-stranded DNA treated in the step 1; and
(3) a step 3 of confirming the presence or absence of an amplification product obtained in the step 2.

16. A reagent for determining cancer, comprising:
S1 nuclease; and
a methylation-sensitive restriction enzyme.

17. A marker for determining cancer, obtained by carrying out the following steps 1 and 2:
(1) a step 1 of treating double-stranded DNA containing an analysis target region with S1 nuclease and a methylation-sensitive restriction enzyme; and
(2) a step 2 of amplifying the analysis target region of the double-stranded DNA treated in the step 1.


SEQUENCE LISTING

<110>  Wako Pure Chemical Industries, Ltd.

<120>  Amplification Method for Methylation DNA, Determination Method
       for Methylation DNA and Determination Method for Cancer

<130>  2055PCT

<150>  JP2016-171550
<151>  2016-09-02

<160>  18

<170>  PatentIn version 3.5

<210>  1
<211>  267
<212>  DNA
<213>  Homo sapiens

<400>  1
caaggtcgga gacaccaggc aattcggaga aggcaggaga gagaagcaga gagggcctgg      60

agggcgagag ggcaaagtgg cgggactgga ggggccgagt gggaattagt gggggcagcc     120

aggccccagg aacggagtgc ggagagattc tgtggtccag tgcgggccgg agggcggtgg     180

aggagccggg ggcgatgccg cggccgggga agagctcgta cagcgaccaa aaaccgccct     240

actcttacat ctcgctgacc gccatgg                                         267


<210>  2
<211>  25
<212>  DNA
<213>  Artificial Sequence

<220>
<223>  oligonucleotide primer

<400>  2
caaggtcgga gacaccaggc aattc                                            25


<210>  3
<211>  24
<212>  DNA
<213>  Artificial Sequence

<220>
<223>  oligonucleotide primer

<400>  3
ccatggcggt cagcgagatg taag                                             24


<210>  4
<211>  433
<212>  DNA
<213>  Canis familiaris

<400>  4

gagctccctt ctcctgacct cgcctctccg ctccaggacc accacggctg cggcgcgggc 60

cggctcggcg cccgagcggg gggcagcctc cgggcgcggc cagcctcgcc tccccggccc 120

tccaggagcg cgagcgccga ctcggacttg aagcgccggg gtcccgcctg tccagtcctt 180

cctcgccgcc cgctgggcct gggcttccct cggaccgaga gaaagcaaga gaggaggccg 240

ggagggaaca cggccctgcg gagcacgcct cggccgccgg ctccagcgcg cggtctcgga 300

ccacctgggg gagccgcgcg gagggcgcgg ggggcgcggg ggcacctggc gcgccccgtc 360

ctccactcgg cgcgcgggtc ggccaccgtc gggcgcacgc tgcgagcggc cgagagcgga 420

gctggccgga gag 433

<210> 5
<211> 22
<212> DNA
<213> Artificial Sequence

<220>
<223> oligonucleotide primer

<400> 5
gagctccctt ctcctgacct cg 22

<210> 6
<211> 22
<212> DNA
<213> Artificial Sequence

<220>
<223> oligonucleotide primer

<400> 6
ctctccggcc agctccgctc tc 22

<210> 7
<211> 29
<212> DNA
<213> Artificial Sequence

<220>
<223> oligonucleotide primer

<400> 7
ggaattagtg ggggcagcca ggccccagg 29

<210> 8
<211> 28
<212> DNA
<213> Artificial Sequence

<220>
<223> oligonucleotide primer

<400> 8
cccaaaaact acccttacca aactaatc 28

<210> 9
<211> 42
<212> DNA
<213> Artificial Sequence

<220>
<223> oligonucleotide primer

<400> 9
ttaccataag cttggaatta gtgggggcag ccaggcccca gg 42


<210> 10
<211> 41
<212> DNA
<213> Artificial Sequence

<220>
<223> oligonucleotide primer

<400> 10
taattaagga tcccccaaaa actaccctta ccaaactaat c 41


<210> 11
<211> 149
<212> DNA
<213> Homo sapiens

<400> 11
ctggaggggc cgagtgggaa ttagtggggg cagccaggcc ccaggaacgg agtgcggaga 60

gattctgtgg tccagtgcgg gccggagggc ggtggaggag ccgggggcga tgccgcggcc 120

ggggaagagc tcgtacagcg accaaaaac 149


<210> 12
<211> 24
<212> DNA
<213> Artificial Sequence

<220>
<223> oligonucleotide primer

<400> 12
ctggaggggc cgagtgggaa ttag 24


<210> 13
<211> 26
<212> DNA
<213> Artificial Sequence

<220>
<223> oligonucleotide primer

<400> 13
gtttttggtc gctgtacgag ctcttc 26

<210>  14
<211>  25
<212>  DNA
<213>  Artificial Sequence

<220>
<223>  oligonucleotide probe

<400>  14
cacagaatct ctccgcactc cgttc                                              25


<210>  15
<211>  149
<212>  DNA
<213>  Homo sapiens

<400>  15
gaagcttgtc atcaatggaa atcccatcac catcttccag gagtgagtgg aagacagaat        60

ggaagaaatg tgctttgggg aggcaactag gatggtgtgg ctcccttggg tatatggtaa       120

ccttgtgtcc ctcaatatgg tcctgtccc                                         149


<210>  16
<211>  25
<212>  DNA
<213>  Artificial Sequence

<220>
<223>  oligonecleotide primer

<400>  16
gaagcttgtc atcaatggaa atccc                                              25


<210>  17
<211>  25
<212>  DNA
<213>  Artificial Sequence

<220>
<223>  oligonecleotide primer

<400>  17
gggacaggac catattgagg gacac                                              25


<210>  18
<211>  25
<212>  DNA
<213>  Artificial Sequence

<220>
<223>  oligonecleotide probe

<400>  18
caactaggat ggtgtggctc ccttg                                              25